# EUROPEAN PATENT APPLICATION

(11) **EP 4 190 326 A1**
(43) Date of publication of application: **07.06.2023**
(21) Application number: 23153650.9
(22) Date of filing: 12.03.2018
(51) Int. Cl.: A61K 31/40, A61K 31/04, A61K 31/496, A61K 39/395, A61P 35/00, A61P 37/00, A61K 9/00, A61K 31/337, A61K 45/06, C07K 16/28, A61P 11/00, A61P 29/00

(54) **COMPOSITIONS OF PLINABULIN AND USE THEREOF**

(30) Priority: 13.03.2017 US 201762470802 P; 24.01.2018 US 201862621528 P
(62) Divisional of application: 18768238.0
(71) Applicant: Beyondspring Pharmaceuticals, Inc., New York, NY 10005 (US)
(72) Inventor: MOHANLAL, Ramon, New York, 10005 (US); HUANG, Lan, New York, 10005 (US); LLOYD, George, Kenneth, New York, 10005 (US)
(74) Representative: Callaghan, Dayle Anne

(57) **Abstract**

Disclosed herein are compositions and methods of preventing and/or treating or reducing immunotherapy mediated adverse events by administering plinabulin. Some embodiments relate to treatment of immunotherapy related inflammation by administering plinabulin. Some embodiments relate to treatment of psoriasis and/or inflammation by administering plinabulin.

## Description

### BACKGROUND

### Field

The present invention relates to the field of chemistry and medicine. More particularly, the present application relates to plinabulin, compositions containing plinabulin and its analogs and use.

### Description of the Related Art

Immunotherapy related adverse events (IRAE) are a frequently observed consequence of immunostimulatory antibody therapy. The IRAEs can be severe and even life-threatening. The IRAEs include autoimmune responses, such as diarrhea, enterocolitis, dermatitis, hypophysitis, panhypopituitarism, rash, pruritis, and other inflammation reactions. Severe IRAEs can result in modification of the drug dosage and less effective treatment or even termination of the cancer treatment. There is a need to develop effective treatment of the IRAEs.

Phosphodiesterases (pdes) are enzymes that hydrolyze and degrade camp. Pde4 is a camp phosphodiesterase widely expressed in hematopoietic cells (e.g. Myeloid, lymphoid), nonhematopoietic cells (e.g. Smooth muscle, keratinocyte, endothelial), and sensory/memory neurons. The four pde4 genes (a, b, c, and d) exhibit distinct target and regulatory properties. Each of these genes can produce multiple protein products due to mrna splice variants, resulting in approximately 19 different pde4 proteins that fall into either short or long isoform categories. Regulation of camp activity is important in many biological processes, and there is a need to develop effective pde4 inhibitors that can be used to treat various diseases.

### SUMMARY

Some embodiments relate to a method of preventing and/or treating an immunotherapy mediated adverse event in a subject, comprising administering an effective amount of plinabulin to the subject in need thereof, wherein the immunotherapy mediated adverse event is selected from the group consisting of pancreatitis, pneumonitis, colitis, hepatitis, nephritis and renal dysfunction, hypothyroidism and hyperthyroidism, uveitis, demyelination, autoimmune neuropathy, adrenal insufficiency, facial and abducens nerve paresis, hypophysitis, diabetic ketoacidosis, hypopituitarism, Guillain-Barré syndrome, myasthenic syndrome, hypothysitis, thyroiditis, type 1 diabetes mellitus, arthritis, exfoliative dermatitis, bullous pemphigoid, myositis, myasthenia gravis, vasculitis, hemolytic anemia, partial seizures arising in a patient with inflammatory foci in brain parenchyma, dermatitis, rash, pruritus, meningitis, sarcoidosis, pericarditis, fatal myocarditis, angiopathy, temporal arteritis, vasculitis, polymyalgia rheumatica, conjunctivitis, blepharitis, episcleritis, scleritis, iritis, leukocytoclastic vasculitis, erythema multiforme, psoriasis, arthritis, autoimmune thyroiditis, neurosensory hypoacusis, autoimmune central neuropathy (encephalitis), myositis, polymyositis, and ocular myositis, and hemolytic anemia.

Some embodiments relate to a method of treating or ameliorating immunotherapy mediated pancreatitis in a subject, comprising administering an effective amount of plinabulin to the subject in need thereof, wherein the immunotherapy comprises administering a PD-1 inhibitor and a CTLA-4 inhibitor.

Some embodiments relate to a method of treating an inflammatory skin or joint disorder in a subject, comprising topically administering an effective amount of plinabulin to the subject in need thereof.

Some embodiments relate to a method of treating an inflammatory disease in a subject, comprising administering an effective amount of plinabulin to the subject in need thereof, wherein the inflammatory disease is selected from the group consisting of chronic articular rheumatism, chronic obstructive pulmonary disease, asthma, ankylosing spondylitis, psoriatic arthritis, sarcoidosis, systemiclupus erythematosus, inflammatory bowel disease, atopic dermatitis, and multiple sclerosis, irritable bowel syndrome, inflammatory bowel disease, and allergic dermatitis.

Some embodiments relate to a method of treating chronic obstructive pulmonary disease or asthma in a subject comprising administering an effective amount of plinabulin to the subject in need thereof through an inhaler.

Some embodiments relate to a method of treating immunotherapy induced inflammation in a subject, comprising administering an effective amount of plinabulin to the subject in need thereof, wherein the subject is administered one or more immune checkpoint inhibitor.

Some embodiments relate to a topical formulation, comprising plinabulin at a concentration effective to inhibit PDE4 activity without reducing vascular proliferation or density.

Some embodiments relate to a topical formulation, comprising plinabulin at a concentration in the range of about 0.1% to about 10% by weight of the total formulation.

### BRIEF DESCRIPTION OF THE DRAWINGS

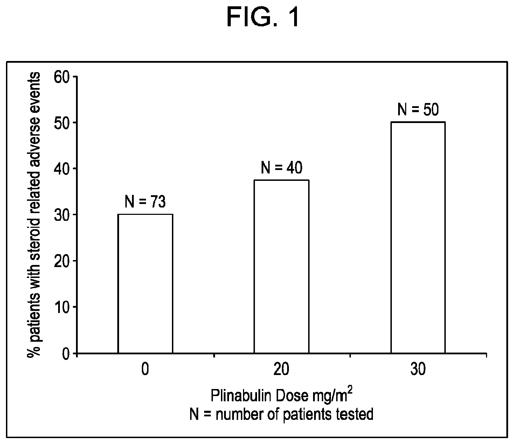

Figure 1 shows the percentage of patients showing steroid related adverse events during treatment with plinabulin.

### DETAILED DESCRIPTION OF THE PREFERRED EMBODIMENT

Plinabulin, (3*Z*,6*Z*)-3-Benzylidene-6-{[5-(2-methyl-2-propanyl)-1*H-*imidazol-4-yl]methylene}-2,5-piperazinedione, is a synthetic analog of the natural compound phenylahistin. Plinabulin can be readily prepared according to methods and procedures detailed in U.S. Patent Nos. 7,064,201 and 7,919,497, which are incorporated herein by reference in their entireties. In some embodiments, Plinabulin can efficiently inhibit PED4 activity. Plinabulin, as described herein, includes pharmaceutically acceptable salts, polymorphs, and solvates thereof.

Plinabulin can be effective in reducing the incidence and severity of IRAEs and when used in combination with the anti-cancer immunotherapy can help achieve a more effective and safe treatment. In addition to treating and preventing IRAEs, Plinabulin can also enhance the immune response during the immunotherapy treatment and achieve a synergistic effect. Plinabulin can target cancer cell and reduce tumor size through immune modulation of the cancer cell and/or tumor microenvironment to promote anti-cancer/anti-tumor immune enhancing effects and act in synergy with the immunotherapy. Therefore, plinabulin not only reduces the risk of immunotherapy related adverse event and improves the safety of the therapy but also promote the effectiveness of the immunotherapy anti-cancer treatment.

Phosphodiesterase 4 (PDE4) is a key enzyme in the degradation of cyclic adenosine monophosphate and is involved in the cytokine production of inflammatory cells, angiogenesis, and the functional properties of other cell types such as keratinocytes. Plinabulin can inhibit the PDE4 activity and have anti-inflammatory properties. Plinabulin can be effective in treating inflammatory disease, including immunotherapy mediated inflammation, and other human chronic inflammatory diseases such as psoriasis and psoriatic arthritis. Plinabulin can be used for treatment of chronic inflammatory diseases such as those of the skin and the joints.

### Definitions

Unless defined otherwise, all technical and scientific terms used herein have the same meaning as is commonly understood by one of ordinary skill in the art to which this disclosure belongs. All patents, applications, published applications, and other publications are incorporated by reference in their entirety. In the event that there is a plurality of definitions for a term herein, those in this section prevail unless stated otherwise.

"Subject" as used herein, means a human or a non-human mammal, e.g., a dog, a cat, a mouse, a rat, a cow, a sheep, a pig, a goat, a non-human primate or a bird, e.g., a chicken, as well as any other vertebrate or invertebrate.

The term "mammal" is used in its usual biological sense. Thus, it specifically includes, but is not limited to, primates, including simians (chimpanzees, apes, monkeys) and humans, cattle, horses, sheep, goats, swine, rabbits, dogs, cats, rodents, rats, mice guinea pigs, or the like.

An "effective amount" or a "therapeutically effective amount" as used herein refers to an amount of a therapeutic agent that is effective to relieve, to some extent, or to reduce the likelihood of onset of, one or more of the symptoms of a disease or condition, and includes curing a disease or condition.

"Treat," "treatment," or "treating," as used herein refers to administering a compound or pharmaceutical composition to a subject for prophylactic and/or therapeutic purposes. The term "prophylactic treatment" refers to treating a subject who does not yet exhibit symptoms of a disease or condition, but who is susceptible to, or otherwise at risk of, a particular disease or condition, whereby the treatment reduces the likelihood that the patient will develop the disease or condition. The term "therapeutic treatment" refers to administering treatment to a subject already suffering from a disease or condition.

The term "pharmaceutically acceptable salt" refers to salts that retain the biological effectiveness and properties of a compound and, which are not biologically or otherwise undesirable for use in a pharmaceutical. In many cases, the compounds disclosed herein are capable of forming acid and/or base salts by virtue of the presence of amino and/or carboxyl groups or groups similar thereto. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Pharmaceutically acceptable salts can also be formed using inorganic and organic bases. Inorganic bases from which salts can be derived include, for example, bases that contain sodium, potassium, lithium, ammonium, calcium, magnesium, iron, zinc, copper, manganese, aluminum, and the like; particularly preferred are the ammonium, potassium, sodium, calcium and magnesium salts. In some embodiments, treatment of the compounds disclosed herein with an inorganic base results in loss of a labile hydrogen from the compound to afford the salt form including an inorganic cation such as Li⁺, Na⁺, K⁺, Mg²⁺ and Ca²⁺ and the like. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. Many such salts are known in the art, as described in WO 87/05297, Johnston et al., published September 11, 1987 (incorporated by reference herein in its entirety).

The term "topical administration" or "topically administer" refers to the application of a pharmaceutical agent to the external surface of the skin, nail, hair, hand or foot, such that the agent crosses the external surface of the skin, nail, hair, hand or foot, and enters the underlying tissues. Topical administration includes application of the composition to intact skin, nail, hair, hand or foot, or to a broken, raw or open wound of skin, nail, hair, hand or foot. Topical administration of a pharmaceutical agent can result in a limited distribution of the agent to the skin and surrounding tissues or, when the agent is removed from the treatment area by the bloodstream, can result in systemic distribution of the agent.

PD-1 is a key immune checkpoint receptor expressed by activated T and B cells and mediates immunosuppression. PD-1 is a member of the CD28 family of receptors, which includes CD28, CTLA-4, ICOS, PD-1, and BTLA. The term "PD-1" as used herein includes human PD-1 (hPD-1), variants, isoforms, and species homologs of hPD-1, and analogs having at least one common epitope with hPD-1.

Various cell surface glycoprotein ligands for PD-1 have been identified, including PD-L1, PD-L2, PD-L3, and PD-L4, that are expressed on antigenpresenting cells as well as many human cancers and have been shown to downregulate T cell activation and cytokine secretion upon binding to PD-1. The term "PD-L1" as used herein includes human PD-L1 (hPD-L1), variants, isoforms, and species homologs of hPD-L1, and analogs having at least one common epitope with hPD-L1. The term "PD-L2" as used herein includes human PD-L2 (hPD-L2), variants, isoforms, and species homologs of hPD-L2, and analogs having at least one common epitope with hPD-L2. The term "PD-L3" as used herein includes human PD-L3 (hPD-L3), variants, isoforms, and species homologs of hPD-L3, and analogs having at least one common epitope with hPD-L3. The term "PD-L4" as used herein includes human PD-L4 (hPD-L4), variants, isoforms, and species homologs of hPD-L4, and analogs having at least one common epitope with hPD-L4.

CTLA-4 (cytotoxic T-lymphocyte-associated protein 4) is a protein receptor that, functioning as an immune checkpoint, downregulates the immune system. CTLA4 is found on the surface of T cells, is also a member of the immunoglobulin (Ig) superfamily; CTLA-4 comprises a single extracellular Ig domain. CTLA-4 transcripts have been found in T cell populations having cytotoxic activity, suggesting that CTLA-4 might function in the cytolytic response.

### Method of Treatment

1. Some embodiments relate to a method of treating or preventing immunotherapy related adverse event in a subject, comprising administering an effective amount of plinabulin to the subject in need thereof, wherein the subject is administered one or more immune checkpoint inhibitor. In some embodiments, the immunotherapy is an anti-cancer immunotherapy. In some embodiments, the subject also receives radiation therapy in addition to the anti-cancer immunotherapy.

Some embodiments relate to a method of treating or preventing immunotherapy induced inflammation in a subject, comprising administering an effective amount of plinabulin to the subject in need thereof, wherein the subject is administered one or more immune checkpoint inhibitor. In some embodiments, the immunotherapy is an anti-cancer immunotherapy.

Some embodiments relate to a method of treating, preventing, or ameliorating immunotherapy mediated adverse event in a subject, comprising administering an effective amount of plinabulin to the subject in need thereof. Immunotherapy mediated adverse event can include, but are not limited to, the adverse events or conditions disclosed in the package inserts of pembrolizumab, nivolumab, atezolizumab, durvalumab, avelumab. ipilimumab and tremelimumab, which have been incorporated herein by reference in their entirety for this purpose.

In some embodiments, the immunotherapy mediated adverse event is selected from the group consisting of pancreatitis, pneumonitis, colitis, hepatitis, nephritis and renal dysfunction, hypothyroidism and hyperthyroidism, uveitis, demyelination, autoimmune neuropathy, adrenal insufficiency, facial and abducens nerve paresis, hypophysitis, diabetic ketoacidosis, hypopituitarism, Guillain-Barré syndrome, myasthenic syndrome, hypothysitis, thyroiditis, type 1 diabetes mellitus, arthritis, exfoliative dermatitis, bullous pemphigoid, myositis, myasthenia gravis, vasculitis, hemolytic anemia, partial seizures arising in a patient with inflammatory foci in brain parenchyma, dermatitis, rash, pruritus, meningitis, sarcoidosis, pericarditis, fatal myocarditis, angiopathy, temporal arteritis, vasculitis, polymyalgia rheumatica, conjunctivitis, blepharitis, episcleritis, scleritis, iritis, leukocytoclastic vasculitis, erythema multiforme, psoriasis, arthritis, autoimmune thyroiditis, neurosensory hypoacusis, autoimmune central neuropathy (encephalitis), myositis, polymyositis, and ocular myositis, and hemolytic anemia. In some embodiments, the immunotherapy mediated adverse event is selected from the group consisting of pancreatitis, pneumonitis, colitis, hepatitis, nephritis, renal dysfunction, hypothyroidism, hyperthyroidism, and uveitis. In some embodiments, the immunotherapy mediated adverse event is pancreatitis. In some embodiments, the immunotherapy is administration of one or more checkpoint inhibitor.

In some embodiments, the immunotherapy related adverse event is selected from fatigue, rash, musculoskeletal pain, pruritus, diarrhea, nausea, asthenia, cough, dyspnea, constipation, decreased appetite, back pain, arthralgia, upper respiratory tract infection, pyrexia, headache, abdominal pain.

In some embodiments, the immunotherapy related adverse event is selected from the group consisting of Pneumonitis, Colitis, Hepatitis, Endocrinopathies, Nephritis and Renal Dysfunction, Skin Adverse Reactions, and Encephalitis. In some embodiments, the immunotherapy related adverse event is selected from infusion reaction, complications of allogenic hematopoietic stem cell transplantation (HSCT), embryo-fetal toxicity. Immune- related pneumonitis: In some embodiments, the immunotherapy related adverse event is pneumonitis. In some embodiments, the immunotherapy related adverse event is colitis. In some embodiments, the immunotherapy related adverse event is hepatitis. In some embodiments, the immunotherapy related adverse event is transaminase or total bilirubin elevation. In some embodiments, the immunotherapy related adverse event is endocrinopathies. In some embodiments, the immunotherapy related adverse event is hypophysitis. In some embodiments, the immunotherapy related adverse event is adrenal insufficiency. In some embodiments, the immunotherapy related adverse event is changes in thyroid function. In some embodiments, the immunotherapy related adverse event is hyperglycemia. In some embodiments, the immunotherapy related adverse event is nephritis and renal dysfunction. In some embodiments, the immunotherapy related adverse event is serum creatinine elevation. In some embodiments, the immunotherapy related adverse event is skin adverse reactions. In some embodiments, the immunotherapy related adverse event is rash. In some embodiments, the immunotherapy related adverse event is Stevens-Johnson syndrome (SJS) and toxic epidermal necrolysis. In some embodiments, the immunotherapy related adverse event is encephalitis. In some embodiments, the immunotherapy related adverse event is changes in neurologic function. In some embodiments, the adverse event is grade 2. In some embodiments, the adverse event is grade 3 or 4. In some embodiments, the adverse event is grade 3. In some embodiments, the adverse event is grade 4. In some embodiments, the adverse event is severe or life threatening.

Some embodiments relate to a method of treating, preventing or ameliorating immunotherapy mediated pancreatitis in a subject, comprising administering an effective amount of plinabulin to the subject in need thereof. Some embodiments relate to a method of treating, preventing or ameliorating immunotherapy mediated Pneumonitis in a subject, comprising administering an effective amount of plinabulin to the subject in need thereof. Some embodiments relate to a method of treating, preventing or ameliorating immunotherapy mediated Colitis in a subject, comprising administering an effective amount of plinabulin to the subject in need thereof. Some embodiments relate to a method of treating, preventing or ameliorating immunotherapy mediated Hepatitis in a subject, comprising administering an effective amount of plinabulin to the subject in need thereof. Some embodiments relate to a method of treating, preventing or ameliorating immunotherapy mediated Endocrinopathies in a subject, comprising administering an effective amount of plinabulin to the subject in need thereof. Some embodiments relate to a method of treating, preventing or ameliorating immunotherapy mediated Nephritis and Renal Dysfunction in a subject, comprising administering an effective amount of plinabulin to the subject in need thereof. Some embodiments relate to a method of treating, preventing or ameliorating immunotherapy mediated Skin Adverse Reactions in a subject, comprising administering an effective amount of plinabulin to the subject in need thereof. Some embodiments relate to a method of treating, preventing or ameliorating immunotherapy mediated Encephalitis in a subject, comprising administering an effective amount of plinabulin to the subject in need thereof. In some embodiments, the immunotherapy comprises administering a PD-1 inhibitor and a CTLA-4 inhibitor. In some embodiments, the immunotherapy comprises administering a PD-1 inhibitor. In some embodiments, the immunotherapy comprises administering nivolumab. In some embodiments, the immunotherapy comprises administering pembrolizumab. In some embodiments, the immunotherapy comprises administering nivolumab in combination with ipilimumab.

In some embodiments, the method described herein further includes identifying a patient at risk of having severe immunotherapy related adverse event. In some embodiments, identifying a patient at risk of having severe immunotherapy related adverse event comprises assessing the patient history (and family history), general physical condition, autoimmune diseases, baseline laboratory tests and radiological exams [mostly computed tomography (CT) scans of the chest, abdomen/pelvis and often brain magnetic resonance imaging (MRI)]. In some embodiments, identifying a patient at risk of having severe immunotherapy related adverse event comprises identifying patients with a history of autoimmune disease, or who are being actively treated for an autoimmune disease. In some embodiments, identifying a patient at risk of having severe immunotherapy related adverse event comprises identifying patients that have had IRAEs on during prior immunotherapy treatment.

In some embodiments, the method described herein comprises identifying a patient having a severe immunotherapy related adverse event. In some embodiments, the method described herein comprises identifying a patient having a grade 3 or 4 immunotherapy related adverse event. In some embodiments, identifying a patient having a severe immunotherapy related adverse event comprises assessing the severity of the skin AE through evaluation by a careful and thorough physical examination of the skin including the mucosal areas, an appreciation of the general patient status (fever, enlarged lymph nodes etc.), and if needed, a biological checkup including a blood cell count, liver and kidney tests. In some embodiments, identifying a patient having a severe immunotherapy related adverse event comprises eliminating the possibility of a dermatological emergency such as drug rash with eosinophilia and systemic symptoms (DRESS), acute febrile neutrophilic dermatosis (Sweet syndrome), Stevens-Johnson syndrome or toxic epidermal necrolysis (TEN). In some embodiments, the IRAE can be graded using the National Cancer Institute Common Toxicity Criteria version 4.0, which is incorporated by reference in its entirety. In some embodiments, the IRAE can be graded using the American Society of Clinical Oncology Clinical Practice Guideline, which is incorporated by reference in its entirety. In some embodiments, the IRAE can be graded using the American Society of Clinical Oncology Clinical Practice Guideline (Management of Toxicities from Immunotherapy: EMSO Clinical Practice Guidelines: Ann Oncol (2017) 28 (suppl 4): iv119-iv142.), which is incorporated by reference in its entirety.

In some embodiments, to gauge the severity of the skin AE, the Common Terminology Criteria for Adverse Events (CTCAE) classification, which is incorporated by reference in its entirety, can be used. Concerning a maculopapular rash, the most frequent event with checkpoint inhibitors, the fourth version of the CTCAE classification includes: Grade 1: macules/papules covering < 10% the body surface area (BSA) with or without symptoms (e.g. pruritus, burning, tightness); Grade 2: macules/papules covering 10%-30% BSA with or without symptoms (e.g. pruritus, burning, tightness); limiting instrumental activities of daily living (ADL); Grade 3: macules/papules covering > 30% BSA with or without associated symptoms; limiting selfcare ADL; and Grade 4: papulopustular rash associated with life-threatening superinfection; Stevens-Johnson syndrome, TEN and bullous dermatitis covering > 30% of BSA and requiring intensive care unit (ICU) admission.

In some embodiments, identifying a patient having a severe immunotherapy related adverse event comprises measuring thyroid-stimulating hormone (TSH), triiodothyronine and thyroxine (FT3, FT4) and anti-thyroid antibodies (Abs) levels of the patient. In some embodiments, identifying a patient having a severe immunotherapy related adverse event comprises monitoring patient's blood glucose levels in order to detect the emergence of DM. In some embodiments, the method described herein includes monitoring during treatment TFTs every cycle ornTFTs 4-6 weeks after cycle 4 (i.e. with restaging CT) for Anti-CTLA4 (including combination with anti-PD-1). In some embodiments, the method described herein includes monitoring during treatment TFTs every cycle for first 3 months, every second cycle thereafter (in case of 2-weekly schedule) when Anti-PD-1/Anti-PD-L1 is used.

In some embodiments, identifying a patient having a severe immunotherapy related adverse event comprises evaluating severe mass effect symptoms, i.e. severe headache, any visual disturbance or Severe hypoadrenalism, i.e. hypotension, severe electrolyte disturbance. In some embodiments, identifying a patient having a severe immunotherapy related adverse event comprises evaluating moderate symptoms, i.e. headache but no visual disturbance or Fatigue/mood alteration but haemodynamically stable, no electrolyte disturbance. In some embodiments, identifying a patient having a severe immunotherapy related adverse event comprises following MRI pituitary protocol or exclude brain metastases) Consider formal visual field assessment (if abnormal patient to inform driver licensing agency).

In some embodiments, identifying a patient having a severe immunotherapy related adverse event comprises assessing the ICPi-related toxicity: management of hepatitis. In some embodiments, to gauge the severity of adverse event, the following standards can be used-Grade 1: ALT or AST > ULN-3x ULN; Grade 2: ALT or AST 3-5x ULN; Grade 3: ALT or AST 5-20x ULN; and Grade 4: ALT or AST > 20x ULN.

In some embodiments, identifying a patient having a severe immunotherapy related adverse event comprises assessing the ICPi-related toxicity: management of diarrhoea and colitis. In some embodiments, a severe (grade 3/4) adverse event includes more than 6 liquid stools per day over baseline or if episodes within 1 h of eating. In some embodiments, a moderate (grade 2) adverse event includes 4- 6 liquid stools per day over baseline or abdominal pain or blood in stool or nausea or nocturnal episodes. In some embodiments, a mild (grade 1) adverse event includes less than 4 liquid stools per day over baseline.

In some embodiments, identifying a patient having a severe immunotherapy related adverse event comprises assessing the ICPi-related toxicity: management of pneumonitis. In some embodiments, a mild (grade 1) adverse event includes radiographic changes only. In some embodiments, a moderate (grade 2) adverse event includes mild /moderate new symptoms, Dyspnoea, cough, chest pain. In some embodiments, a severe (grade 3/4) adverse event includes severe new symptoms; New/worsening hypoxia; Life threatening; Difficulty in breathing, ARDS.

In some embodiments, the one or more checkpoint inhibitor is selected from the group consisting of a PD-1 inhibitor, a PD-L1 inhibitor, a PD-L2 inhibitor, a PD-L3 inhibitor, a PD-L4 inhibitor, a CTLA-4 inhibitor, a LAG3 inhibitor, a B7-H3 inhibitor, a B7-H4 inhibitor, a KIR inhibitor and a TIM3 inhibitor. In some embodiments, the immune checkpoint inhibitor is a PD-1 inhibitor. In some embodiments, the immune checkpoint inhibitor is a PD-L1 inhibitor. In some embodiments, the immune checkpoint inhibitor is a CTLA-4 inhibitor.

In some embodiments, the subject is administered a first immune checkpoint inhibitor and a second immune checkpoint inhibitor, and wherein the first immune checkpoint inhibitor is different from the second immune checkpoint inhibitor. In some embodiments, the first and the second immune checkpoint inhibitor is independently an inhibitor selected from the group consisting of a PD-1 inhibitor, a PD-L1 inhibitor, a PD-L2 inhibitor, a PD-L3 inhibitor, a PD-L4 inhibitor, a CTLA-4 inhibitor, a LAG3 inhibitor, a B7-H3 inhibitor, a B7-H4 inhibitor, a KIR inhibitor and a TIM3 inhibitor. In some embodiments, the first checkpoint inhibitor is a PD-1 inhibitor or a PD-L1 inhibitor, and the second checkpoint inhibitor is a CTLA-4 inhibitor.

In some embodiments, the immunotherapy is a monotherapy, and wherein the monotherapy comprises administering a single checkpoint inhibitor selected from the group consisting of a PD-1 inhibitor, a PD-L1 inhibitor, a PD-L2 inhibitor, a PD-L3 inhibitor, a PD-L4 inhibitor, a CTLA-4 inhibitor, a LAG3 inhibitor, a B7-H3 inhibitor, a B7-H4 inhibitor, a KIR inhibitor and a TIM3 inhibitor. In some embodiments, the checkpoint inhibitor is PD-1 inhibitor. In some embodiments, the checkpoint inhibitor is a PD-L1 inhibitor. In some embodiments, the checkpoint inhibitor is a CTLA-4 inhibitor.

In some embodiments, the immunotherapy is a combination therapy, and wherein the combination therapy comprises administering two or more checkpoint inhibitors selected from the group consisting of a PD-1 inhibitor, a PD-L1 inhibitor, a PD-L2 inhibitor, a PD-L3 inhibitor, a PD-L4 inhibitor, a CTLA-4 inhibitor, a LAG3 inhibitor, a B7-H3 inhibitor, a B7-H4 inhibitor, a KIR inhibitor and a TIM3 inhibitor. In some embodiments, the combination therapy comprises administering a PD-L1 inhibitor and a CTLA-4 inhibitor.

In some embodiments, the PD-1 or PD-L1 inhibitor is selected from the group consisting of pembrolizumab, nivolumab, atezolizumab, durvalumab, and avelumab. In some embodiments, the PD-1 inhibitor is nivolumab or pembrolizumab,. In some embodiments, the PD-L1 inhibitor is atezolizumab, durvalumab, or avelumab. In some embodiments, the CTLA-4 inhibitor is ipilimumab or tremelimumab. In some embodiments, the checkpoint inhibitor is pembrolizumab. In some embodiments, the checkpoint inhibitor is nivolumab.

In some embodiments, the amount of plinabulin used to treat the indications described herein is effective to inhibit PDE4 activity without reducing avascular proliferation or density.

In some embodiments, the immunotherapy is used for treating or preventing Melanoma, Non-Small Cell Lung Cancer (NSCLC), Head and Neck Squamous Cell Cancer (HNSCC), Classical Hodgkin Lymphoma (cHL), Urothelial Carcinoma, Microsatellite Instability-High Cancer (MSI-H Cancer), Gastric Cancer, advanced renal cell carcinoma, metastatic squamous cell carcinoma of the head and neck (SCCHN), metastatic colorectal cancer, hepatocellular carcinoma (HCC), or microsatellite instability-high (MSI-H) or mismatch repair deficient (dMMR) metastatic colorectal cancer (CRC). In some embodiments, the immunotherapy is used for treating or preventing Melanoma, Non-Small Cell Lung Cancer (NSCLC), Head and Neck Squamous Cell Cancer (HNSCC), Classical Hodgkin Lymphoma (cHL), Urothelial Carcinoma, Microsatellite Instability-High Cancer (MSI-H Cancer), or Gastric Cancer.

Some embodiments relate to treatment of inflammatory disease or condition in a subject, comprising administering plinabulin to the subject in need thereof, with the proviso that the inflammatory disease or condition is not rheumatoid arthritis, chronic articular rheumatism, psoriasis, diabetic retinopathy, neovascular glaucoma, retinopathy of prematurity, macular degeneration, corneal graft rejection, retrolental fibroplasia, rubeosis, capillary proliferation in atherosclerotic plaques, or osteoporosis.

In some embodiments, the plinabulin is administered at a dose in the range of about 1-50 mg/m² of the body surface area. In some embodiments, the plinabulin is administered at a dose in the range of about 5 to about 50 mg/m² of the body surface area. In some embodiments, the plinabulin is administered at a dose in the range of about 20 to about 40 mg/m² of the body surface area. In some embodiments, the plinabulin is administered at a dose in the range of about 15 to about 30 mg/m² of the body surface area. In some embodiments, the plinabulin is administered at a dose in the range of about 0.5-1, 0.5-2, 0.5-3, 0.5-4, 0.5-5, 0.5-6, 0.5-7, 0.5-8, 0.5-9, 0.5-10, 0.5-11, 0.5-12, 0.5-13, 0.5-13.75, 0.5-14, 0.5-15, 0.5-16, 0.5-17, 0.5-18, 0.5-19, 0.5-20, 0.5-22.5, 0.5-25, 0.5-27.5, 0.5-30, 1-2, 1-3, 1-4, 1-5, 1-6, 1-7, 1-8, 1-9, 1-10, 1-11, 1-12, 1-13, 1-13.75, 1-14, 1-15, 1-16, 1-17, 1-18, 1-19, 1-20, 1-22.5, 1-25, 1-27.5, 1-30, 1.5-2, 1.5-3, 1.5-4, 1.5-5, 1.5-6, 1.5-7, 1.5-8, 1.5-9, 1.5-10, 1.5-11, 1.5-12, 1.5-13, 1.5-13.75, 1.5-14, 1.5-15, 1.5-16, 1.5-17, 1.5-18, 1.5-19, 1.5-20, 1.5-22.5, 1.5-25, 1.5-27.5, 1.5-30, 2.5-2, 2.5-3, 2.5-4, 2.5-5, 2.5-6, 2.5-7, 2.5-8, 2.5-9, 2.5-10, 2.5-11, 2.5-12, 2.5-13, 2.5-13.75, 2.5-14, 2.5-15, 2.5-16, 2.5-17, 2.5-18, 2.5-19, 2.5-20, 2.5-22.5, 2.5-25, 2.5-27.5, 2.5-30, 2.5-7.5, 3-4, 3-5, 3-6, 3-7, 3-8, 3-9, 3-10, 3-11, 3-12, 3-13, 3-13.75, 3-14, 3-15, 3-16, 3-17, 3-18, 3-19, 3-20, 3-22.5, 3-25, 3-27.5, 3-30, 3.5- 6.5, 3.5-13.75, 3.5-15, 2.5-17.5, 4-5, 4-6, 4-7, 4-8, 4-9, 4-10, 4-11, 4-12, 4-13, 4-13.75, 4-14, 4-15, 4-16, 4-17, 4-18, 4-19, 4-20, 4-22.5, 4-25, 4-27.5, 4-30, 5-6, 5-7, 5-8, 5-9, 5-10, 5-11, 5-12, 5-13, 5-13.75, 5-14, 5-15, 5-16, 5-17, 5-18, 5-19, 5-20, 5-22.5, 5-25, 5-27.5, 5-30, 6-7, 6-8, 6-9, 6-10, 6-11, 6-12, 6-13, 6-13.75, 6-14, 6-15, 6-16, 6-17, 6-18, 6-19, 6-20, 6-22.5, 6-25, 6-27.5, 6-30, 7-8, 7-9, 7-10, 7-11, 7-12, 7-13, 7-13.75, 7-14, 7-15, 7-16, 7-17, 7-18, 7-19, 7-20, 7-22.5, 7-25, 7-27.5, 7-30, 7.5-12.5, 7.5-13.5, 7.5-15, 8-9, 8-10, 8-11, 8-12, 8-13, 8-13.75, 8-14, 8-15, 8-16, 8-17, 8-18, 8-19, 8-20, 8-22.5, 8-25, 8-27.5, 8-30, 9-10, 9-11, 9-12, 9-13, 9-13.75, 9-14, 9-15, 9-16, 9-17, 9-18, 9-19, 9-20, 9-22.5, 9-25, 9-27.5, 9-30, 10-11, 10-12, 10-13, 10-13.75, 10-14, 10-15, 10-16, 10-17, 10-18, 10-19, 10-20, 10-22.5, 10-25, 10-27.5, 10-30, 11.5-15.5, 12.5-14.5, 7.5-22.5, 8.5-32.5, 9.5-15.5, 15.5-24.5, 5-35, 17.5-22.5, 22.5-32.5, 25-35, 25.5-24.5, 27.5-32.5, 2-20, t 2.5-22.5, or 9.5-21.5 mg/m², of the body surface area. In some embodiments, the plinabulin is administered at a dose of about 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 32, 33, 34, 35, 36, 37, 38, 39, 40 mg/m² of the body surface area. In some embodiments, the plinabulin is administered at a dose less than about 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 32, 33, 34, 35, 36, 37, 38, 39, 40 mg/m² of the body surface area. In some embodiments, the plinabulin is administered at a dose greater than about 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 32, 33, 34, 35, 36, 37, 38, 39, 40, 41, 42, 43, 44, 45, 46, 47, 48, 49, 50 mg/m² of the body surface area. In some embodiments, the plinabulin is administered at a dose of about 10, 13.5, 20, or 30 mg/m² of the body surface area. In some embodiments, the plinabulin is administered at a dose of about 20 mg/m² of the body surface area.

In some embodiments, the plinabulin dose is about 5 mg - 100 mg, or about 10 mg - 80 mg. In some embodiments, the plinabulin dose is about 15 mg - 100 mg, or about 20 mg - 80 mg. In some embodiments, the plinabulin is administered at a dose in the range of about 15 mg - 60 mg. In some embodiments, the plinabulin dose is about 0.5 mg - 3 mg, 0.5 mg -2 mg, 0.75 mg - 2 mg, 1 mg - 10 mg, 1.5 mg - 10 mg, 2 mg - 10 mg, 3 mg - 10 mg, 4 mg - 10 mg, 1 mg - 8 mg, 1.5 mg - 8 mg, 2 mg - 8 mg, 3 mg - 8 mg, 4 mg - 8 mg, 1 mg - 6 mg, 1.5 mg - 6 mg, 2 mg - 6 mg, 3 mg - 6 mg, or about 4 mg - 6 mg. In some embodiments, the plinabulin administered is about 2 mg - 6 mg or 2 mg - 4.5 mg. In some embodiments, the plinabulin administered is about 5 mg-7.5 mg, 5 mg-9 mg, 5 mg-10 mg, 5 mg-12mg, 5mg-14mg, 5mg-15 mg, 5 mg-16 mg, 5 mg-18 mg, 5 mg-20 mg, 5 mg-22 mg, 5 mg-24 mg, 5 mg-26 mg, 5 mg-28mg, 5mg-30mg, 5mg-32mg, 5mg-34mg, 5mg-36mg, 5mg-38mg, 5mg-40mg, 5mg-42mg, 5mg-44mg, 5mg-46mg, 5mg-48mg, 5mg-50mg, 5mg-52mg, 5mg-54mg, 5mg-56mg, 5mg-58mg, 5mg-60mg, 7 mg-7.7 mg, 7 mg-9 mg, 7 mg-10 mg, 7 mg-12mg, 7mg-14mg, 7mg-15 mg, 7 mg-16 mg, 7 mg-18 mg, 7 mg-20 mg, 7 mg-22 mg, 7 mg-24 mg, 7 mg-26 mg, 7 mg-28mg, 7mg-30mg, 7mg-32mg, 7mg-34mg, 7mg-36mg, 7mg-38mg, 7mg-40mg, 7mg-42mg, 7mg-44mg, 7mg-46mg, 7mg-48mg, 7mg-50mg, 7mg-52mg, 7mg-54mg, 7mg-56mg, 7mg-58mg, 7mg-60mg, 9 mg-10 mg, 9 mg-12mg, 9mg-14mg, 9mg-15 mg, 9 mg-16 mg, 9 mg-18 mg, 9 mg-20 mg, 9 mg-22 mg, 9 mg-24 mg, 9 mg-26 mg, 9 mg-28mg, 9mg-30mg, 9mg-32mg, 9mg-34mg, 9mg-36mg, 9mg-38mg, 9mg-40mg, 9mg-42mg, 9mg-44mg, 9mg-46mg, 9mg-48mg, 9mg-50mg, 9mg-52mg, 9mg-54mg, 9mg-56mg, 9mg-58mg, 9mg-60mg, 10 mg-12mg, 10mg-14mg, 10mg-15 mg, 10 mg-16 mg, 10 mg-18 mg, 10 mg-20 mg, 10 mg-22 mg, 10 mg-24 mg, 10 mg-26 mg, 10 mg-28mg, 10mg-30mg, 10mg-32mg, 10mg-34mg, 10mg-36mg, 10mg-38mg, 10mg-40mg, 10mg-42mg, 10mg-44mg, 10mg-46mg, 10mg-48mg, 10mg-50mg, 10mg-52mg, 10mg-54mg, 10mg-56mg, 10mg-58mg, 10mg-60mg, 12mg-14mg, 12mg-15 mg, 12 mg-16 mg, 12 mg-18 mg, 12 mg-20 mg, 12 mg-22 mg, 12 mg-24 mg, 12 mg-26 mg, 12 mg-28mg, 12mg-30mg, 12mg-32mg, 12mg-34mg, 12mg-36mg, 12mg-38mg, 12mg-40mg, 12mg-42mg, 12mg-44mg, 12mg-46mg, 12mg-48mg, 12mg-50mg, 12mg-52mg, 12mg-54mg, 12mg-56mg, 12mg-58mg, 12mg-60mg, 15 mg-16 mg, 15 mg-18 mg, 15 mg-20 mg, 15 mg-22 mg, 15 mg-24 mg, 15 mg-26 mg, 15 mg-28mg, 15mg-30mg, 15mg-32mg, 15mg-34mg, 15mg-36mg, 15mg-38mg, 15mg-40mg, 15mg-42mg, 15mg-44mg, 15mg-46mg, 15mg-48mg, 15mg-50mg, 15mg-52mg, 15mg-54mg, 15mg-56mg, 15mg-58mg, 15mg-60mg, 17 mg-18 mg, 17 mg-20 mg, 17 mg-22 mg, 17 mg-24 mg, 17 mg-26 mg, 17 mg-28mg, 17mg-30mg, 17mg-32mg, 17mg-34mg, 17mg-36mg, 17mg-38mg, 17mg-40mg, 17mg-42mg, 17mg-44mg, 17mg-46mg, 17mg-48mg, 17mg-50mg, 17mg-52mg, 17mg-54mg, 17mg-56mg, 17mg-58mg, 17mg-60mg, 20 mg-22 mg, 20 mg-24 mg, 20 mg-26 mg, 20 mg-28mg, 20mg-30mg, 20mg-32mg, 20mg-34mg, 20mg-36mg, 20mg-38mg, 20mg-40mg, 20mg-42mg, 20mg-44mg, 20mg-46mg, 20mg-48mg, 20mg-50mg, 20mg-52mg, 20mg-54mg, 20mg-56mg, 20mg-58mg, 20mg-60mg, 22 mg-24 mg, 22 mg-26 mg, 22 mg-28mg, 22mg-30mg, 22mg-32mg, 22mg-34mg, 22mg-36mg, 22mg-38mg, 22mg-40mg, 22mg-42mg, 22mg-44mg, 22mg-46mg, 22mg-48mg, 22mg-50mg, 22mg-52mg, 22mg-54mg, 22mg-56mg, 22mg-58mg, 22mg-60mg, 25 mg-26 mg, 25 mg-28mg, 25mg-30mg, 25mg-32mg, 25mg-34mg, 25mg-36mg, 25mg-38mg, 25mg-40mg, 25mg-42mg, 25mg-44mg, 25mg-46mg, 25mg-48mg, 25mg-50mg, 25mg-52mg, 25mg-54mg, 25mg-56mg, 25mg-58mg, 25mg-60mg, 27 mg-28mg, 27mg-30mg, 27mg-32mg, 27mg-34mg, 27mg-36mg, 27mg-38mg, 27mg-40mg, 27mg-42mg, 27mg-44mg, 27mg-46mg, 27mg-48mg, 27mg-50mg, 27mg-52mg, 27mg-54mg, 27mg-56mg, 27mg-58mg, 27mg-60mg, 30mg-32mg, 30mg-34mg, 30mg-36mg, 30mg-38mg, 30mg-40mg, 30mg-42mg, 30mg-44mg, 30mg-46mg, 30mg-48mg, 30mg-50mg, 30mg-52mg, 30mg-54mg, 30mg-56mg, 30mg-58mg, 30mg-60mg, 33mg-34mg, 33mg-36mg, 33mg-38mg, 33mg-40mg, 33mg-42mg, 33mg-44mg, 33mg-46mg, 33mg-48mg, 33mg-50mg, 33mg-52mg, 33mg-54mg, 33mg-56mg, 33mg-58mg, 33mg-60mg, 36mg-38mg, 36mg-40mg, 36mg-42mg, 36mg-44mg, 36mg-46mg, 36mg-48mg, 36mg-50mg, 36mg-52mg, 36mg-54mg, 36mg-56mg, 36mg-58mg, 36mg-60mg, 40mg-42mg, 40mg-44mg, 40mg-46mg, 40mg-48mg, 40mg-50mg, 40mg-52mg, 40mg-54mg, 40mg-56mg, 40mg-58mg, 40mg-60mg, 43mg-46mg, 43mg-48mg, 43mg-50mg, 43mg-52mg, 43mg-54mg, 43mg-56mg, 43mg-58mg, 42mg-60mg, 45mg-48mg, 45mg-50mg, 45mg-52mg, 45mg-54mg, 45mg-56mg, 45mg-58mg, 45mg-60mg, 48mg-50mg, 48mg-52mg, 48mg-54mg, 48mg-56mg, 48mg-58mg, 48mg-60mg, 50mg-52mg, 50mg-54mg, 50mg-56mg, 50mg-58mg, 50mg-60mg, 52mg-54mg, 52mg-56mg, 52mg-58mg, or 52mg-60mg. In some embodiments, the plinabulin dose is greater than about 0.5mg, 1mg, 1.5 mg, 2 mg, 3 mg, 4 mg, 5 mg, 6 mg, 7 mg, 8 mg, 9 mg, about 10 mg, about 12.5 mg, about 13.5 mg, about 15 mg, about 17.5 mg, about 20 mg, about 22.5 mg, about 25 mg, about 27 mg, about 30 mg, or about 40 mg. In some embodiments, the plinabulin dose is about less than about 1mg, 1.5 mg, 2 mg, 3 mg, 4 mg, 5 mg, 6 mg, 7 mg, 8 mg, 9 mg, about 10 mg, about 12.5 mg, about 13.5 mg, about 15 mg, about 17.5 mg, about 20 mg, about 22.5 mg, about 25 mg, about 27 mg, about 30 mg, about 40 mg, or about 50 mg.

The actual dose of the checkpoint inhibitor described herein depends on the specific compound, and on the condition to be treated; the selection of the appropriate dose is well within the knowledge of the skilled artisan. In some embodiments, the method described herein comprises administering a checkpoint inhibitor at a dose in the range of from about 0.01 mg/kg to about 250 mg/kg of body weight, from about 0.1 mg/kg to about 200 mg/kg of body weight, from about 0.25 mg/kg to about 120 mg/kg of body weight, from about 0.5 mg/kg to about 70 mg/kg of body weight, from about 1.0 mg/kg to about 50 mg/kg of body weight, from about 1.0 mg/kg to about 15 mg/kg of body weight, from about 2.0 mg/kg to about 15 mg/kg of body weight, from about 3.0 mg/kg to about 12 mg/kg of body weight, or from about 5.0 mg/kg to about 10 mg/kg of body weight. In some embodiments, the method described herein comprises administering a checkpoint inhibitor at a dose in the range of 0.5-1, 0.5-2, 0.5-3, 0.5-4, 0.5-5, 0.5-6, 0.5-7, 0.5-8, 0.5-9, 0.5-10, 1-2, 1-3, 1-4, 1-5, 1-6, 1-7, 1-8, 1-9, 1-10, 1-20, 1-30, 1-40, 1-50, 1-60, 1-70, 1-80, 1-90, 1-100, 2.5-5, 2.5-10, 2.5-20, 2.5-30, 2.5-40, 2.5-50, 2.5-60, 2.5-70, 2.5-80, 2.5-90, 2.5-100, 3-5, 3-10, 3-20, 3-30, 3-40, 3-50, 3-60, 3-70, 3-80, 3-90, 3-100, 5-10, 5-20, 5-30, 5-40, 5-50,5-60, 5-70, 5-80, 5-90, 5-100, 7.5-10, 7.5-20, 7.5-30, 7.5-40, 7.5-50, 7.5-60, 7.5-70, 7.5-80, 7.5-90, 7.5-100, 10-10, 10-20, 10-30, 10-40, 10-50, 10-60, 10-70, 10-80, 10-90, 10-100, 10-150, 10-200, 20-30, 20-40, 20-50, 20-60, 20-70, 20-80, 20-90, 20-100, 20-150, 20-200, 30^10, 30-50, 30-60, 30-70, 30-80, 30-90, 30-100, 30-150, 30-200, 40-50, 40-60, 40-70, 40-80, 40-90, 40-100, 40-150, 40-200, 40-300, 50-60, 50-70, 50-80, 50-90, 50-100, 50-150, 50-200, 50-250, 50-300, 60-80, 60-100, 60-150, 60-200, 70-100, 70-150, 70-200, 70-250, 70-300, 80-100, 80-150, 80-200, 80-250, 80-300, 90-100, 90-150, 90-200, 90-250, 90-300, 90-350, 90-400, 100-150, 100-200, 100-250, 100-300, 100-350, or 100-400 mg/kg of body weight. In some embodiments, the checkpoint inhibitor described herein may be administered at a dose of about 0.1, 0.25, 0.5, 1, 2, 3, 4, 5, 6, 7, 8, 9, 10, 11, 12, 13, 14, 15 ,16, 17, 18,19, 20, 22.5, 25, 27.5, 30, 35, 40, 45, 50, 55, 60, 65, 70, 75, 80, 85, 90, 95, or 100 mg/kg of body weight. In some embodiments, the method described herein comprises administering a checkpoint inhibitor at a dose of about 3 mg/kg. In some embodiments, the method described herein comprises administering a checkpoint inhibitor at a dose of about 3 mg/kg every three weeks for a total of four doses.

In some embodiments, the checkpoint inhibitor is administered at an amount of about 0.5-1, 0.5-2, 0.5-3, 0.5-4, 0.5-5, 0.5-6, 0.5-7, 0.5-8, 0.5-9, 0.5-10, .2.5-3, 2.5-4, 2.5-5, 2.5-6, 2.5-7, 2.5-8, 2.5-9, 2.5-10,3-10, 5-10, 1-10, 1-20, 1-30, 1-40, 1-50, 1-60, 1-70, 1-80, 1-90, 1-100, 2.5-10, 2.5-20, 2.5-30, 2.5-40, 2.5-50, 2.5-60, 2.5-70, 2.5-80, 2.5-90, 2.5-100, 5-10, 5-20, 5-30, 5-40, 5-50,5-60, 5-70, 5-80, 5-90, 5-100, 7.5-10, 7.5-20, 7.5-30, 7.5-40, 7.5-50, 7.5-60, 7.5-70, 7.5-80, 7.5-90, 7.5-100, 10-10, 10-20, 10-30, 10-40, 10-50, 10-60, 10-70, 10-80, 10-90, 10-100, 10-150, 10-200, 20-30, 20-40, 20-50, 20-60, 20-70, 20-80, 20-90, 20-100, 20-150, 20-200, 30-40, 30-50, 30-60, 30-70, 30-80, 30-90, 30-100, 30-150, 30-200, 40-50, 40-60, 40-70, 40-80, 40-90, 40-100, 40-150, 40-200, 40-300, 50-60, 50-70, 50-80, 50-90, 50-100, 50-150, 50-200, 50-250, 50-300, 60-80, 60-100, 60-150, 60-200, 70-100, 70-150, 70-200, 70-250, 70-300, 70-500, 70-750, 70-1000, 70-1500, 70-2000, 70-3000, 80-100, 80-150, 80-200, 80-250, 80-300, 80-500, 80-750, 80-1000, 80-1500, 80-2000, 80-3000, 90-100, 90-150, 90-200, 90-250, 90-300, 90-350, 90-400, 90-500, 90-750, 90-1000, 90-1500, 90-2000, 90-3000, 100-150, 100-200, 100-250, 100-300, 100-350, 100-400, 100-500, 100-600, 100-700, 100-800, 100-900, 100-1000, 100-1500, 100-2000, 100-2500, 100-3000, 100-3500, 100-4000, 200-500, 200-700, 200-1000, 200-1500, 200-2000, 200-2500, 200-3000, 200-3500, 200-4000, 500-1000, 500-1500, 500-2000, 500-2500, 500-3000, 500-3500, or 500-4000 mg per dose. In some embodiments, the checkpoint inhibitor is administered at an amount of about 0.1, 0.2, 0.3, 0.4, 0.5, 0.6, 0.7, 0.8, 0.9, 1, 1.25, 1.5, 1.75, 2, 2.25, 2.5, 2.75, 3, 3.25, 3.5, 3.75, 4, 4.5, 5, 5.5, 6, 6.5, 7, 7.5, 8, 8.5, 9, 9.5, 10, 12.5, 15, 17.5, 20, 22.5, 25, 27.5, 30, 40, 50, 60, 70, 80, 90, 100, 150, 200, 250, 300, 350,400, 450, 500, 550, 600, 650, 700, 750, 800, 850, 900, 1000, 1100, 1200, 1250, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, or 5000 mg per dose. In some embodiments, the checkpoint inhibitor is administered at an amount of about 25 mg, 50 mg, or 100 mg per dose.

In some embodiments, the method described herein comprises administering an inhibitor of PD-1 at a dose in the range of about 0.5-1, 0.5-2, 0.5-3, 0.5-4, 0.5-5, 0.5-6, 0.5-7, 0.5-8, 0.5-9, 0.5-10, 1-2, 1-3, 1-4, 1-5, 1-6, 1-7, 1-8, 1-9, 1-10, 1-20, 1-30, 1-40, 1-50, 1-60, 1-70, 1-80, 1-90, 1-100, 2-3, 2-4, 2-5, 2-6, 2-7, 2-8, 2-9, 2-10, 2-20, 2-30, 2-40, 2-50, 2-60, 2-70, 2-80, 2-90, 2-100, 2.5-3, 2.5-3.5, 2.5-4, 2.5-5, 2.5-6, 2.5-7, 2.5-9, 2.5-10, 3-4, 3-5, 3-6, 3-7, 3-8, 3-9, 3-10, 5-10, 5-20, 5-30, 5-40, 5-50,5-60, 5-70, 5-80, 5-90, 5-100, 7.5-10, 7.5-20, 7.5-30, 7.5-40, 7.5-50, 7.5-60, 7.5-70, 7.5-80, 7.5-90, 7.5-100, 10-10, 10-20, 10-30, 10-40, 10-50, 10-60, 10-70, 10-80, 10-90, 10-100, 10-150, 10-200, 20-30, 20-40, 20-50, 20-60, 20-70, 20-80, 20-90, 20-100, 20-150, 20-200, 30-40, 30-50, 30-60, 30-70, 30-80, 30-90, 30-100, 30-150, 30-200, 40-50, 40-60, 40-70, 40-80, 40-90, 40-100, 40-150, 40-200, 40-300, 50-60, 50-70, 50-80, 50-90, 50-100, 50-150, 50-200, 50-250, 50-300, 60-80, 60-100, 60-150, 60-200, 70-100, 70-150, 70-200, 70-250, 70-300, 80-100, 80-150, 80-200, 80-250, 80-300, 90-100, 90-150, 90-200, 90-250, 90-300, 90-350, 90-400, 100-150, 100-200, 100-250, 100-300, 100-350, or 100-400 mg/kg of the body weight. In some embodiments, the method described herein comprises administering the inhibitor of PD-1 at a dose of about 0.1, 0.2, 0.3, 0.4, 0.5, 0.6, 0.7, 0.8, 0.9, 1, 1.25, 1.5, 1.75, 2, 2.25, 2.5, 2.75, 3, 3.25, 3.5, 3.75, 4, 4.5, 5, 5.5, 6, 6.5, 7, 7.5, 8, 8.5, 9, 9.5, 10, 12.5, 15, 17.5, 20, 22.5, 25, 27.5, 30, 40, 50, 60, 70, 80, 90 or 100 mg/kg of the body weight. In some embodiments, the inhibitor of PD-1 is administered at a dose of about 3 mg/kg. In some embodiments, the inhibitor of PD-1 is administered at a dose of about 2 mg/kg.

In some embodiments, the PD-1 inhibitor is administered at an amount of about 1-10, 1-20, 1-30, 1-40, 1-50, 1-60, 1-70, 1-80, 1-90, 1-100, 1-150, 1-200, 1-250, 1-300, 1-500, 2.5-3, 2.5-4, 2.5-5, 2.5-6, 2.5-7, 2.5-8, 2.5-9, 2.5-10, 2.5-20, 2.5-30, 2.5-40, 2.5-50, 2.5-60, 2.5-70, 2.5-80, 2.5-90, 2.5-100, 2.5-200, 2.5-250, 2.5-300, 2.5-500, 3-10, 3-20, 3-30, 3-40, 3-50, 3-60, 3-70, 3-80, 3-90, 3-100, 3-200, 3-250, 3-300, 3-500, 5-10, 5-10, 5-20, 5-30, 5-40, 5-50,5-60, 5-70, 5-80, 5-90, 5-100, 7.5-10, 7.5-20, 7.5-30, 7.5-40, 7.5-50, 7.5-60, 7.5-70, 7.5-80, 7.5-90, 7.5-100, 10-10, 10-20, 10-30, 10-40, 10-50, 10-60, 10-70, 10-80, 10-90, 10-100, 10-150, 10-200, 20-30, 20-40, 20-50, 20-60, 20-70, 20-80, 20-90, 20-100, 20-150, 20-200, 30-40, 30-50, 30-60, 30-70, 30-80, 30-90, 30-100, 30-150, 30-200, 40-50, 40-60, 40-70, 40-80, 40-90, 40-100, 40-150, 40-200, 40-300, 50-60, 50-70, 50-80, 50-90, 50-100, 50-150, 50-200, 50-250, 50-300, 60-80, 60-100, 60-150, 60-200, 70-100, 70-150, 70-200, 70-250, 70-300, 70-500, 70-750, 70-1000, 70-1500, 70-2000, 70-3000, 80-100, 80-150, 80-200, 80-250, 80-300, 80-500, 80-750, 80-1000, 80-1500, 80-2000, 80-3000, 90-100, 90-150, 90-200, 90-250, 90-300, 90-350, 90-400, 90-500, 90-750, 90-1000, 90-1500, 90-2000, 90-3000, 100-150, 100-200, 100-250, 100-300, 100-350, 100-400, 100-500, 100-600, 100-700, 100-800, 100-900, 100-1000, 100-1500, 100-2000, 100-2500, 100-3000, 100-3500, 100-4000, 200-500, 200-700, 200-1000, 200-1500, 200-2000, 200-2500, 200-3000, 200-3500, 200-4000, 500-1000, 500-1500, 500-2000, 500-2500, 500-3000, 500-3500, or 500-4000 mg per dose. In some embodiments, the PD-1 inhibitor is administered at an amount of about 10-30, 10-50, 10-80, 10-100, 10-125, 10-150, 10-175, 10-200, 10-250, 10-300, 10-400, 20-50, 20-100, 20-125, 20-150, 20-175, 20-200, 20-250, 20-300, 20-400, 30-50, 30-80, 30-100, 30-125, 30-150, 30-175, 30-200, 30-250, 30-300, 30-400, 40-50, 40-80, 40-100, 40-125, 40-150, 40-175, 40-200, 40-250, 40-300, 40-400, 50-80, 50-100, 50-125, 50-150, 50-175, 50-200, 50-250, 50-300, or 50-400 mg per dose. In some embodiments, the PD-1 inhibitor (e.g., nivolumab or pembrolizumab) is administered at an amount of about 50-350 mg per dose, about 100-300 mg per dose, or about 150-250 mg per dose. In some embodiments, the PD-1 inhibitor (e.g., nivolumab or pembrolizumab) is administered at an amount of about 200 mg per dose. In some embodiments, the PD-1 inhibitor (e.g., nivolumab or pembrolizumab) is administered at an amount of about 240 mg per dose.

In some embodiments, the method described herein comprises administering an inhibitor of PD-L1 at a dose in the range of about 0.5-1, 0.5-2, 0.5-3, 0.5-4, 0.5-5, 0.5-6, 0.5-7, 0.5-8, 0.5-9, 0.5-10, 1-2, 1-3, 1-4, 1-5, 1-6, 1-7, 1-8, 1-9, 1-10, 1-20, 1-30, 1-40, 1-50, 1-60, 1-70, 1-80, 1-90, 1-100, 2-3, 2-4, 2-5, 2-6, 2-7, 2-8, 2-9, 2-10, 2-20, 2-30, 2-40, 2-50, 2-60, 2-70, 2-80, 2-90, 2-100, 2.5-3, 2.5-3.5, 2.5-4, 2.5-5, 2.5-6, 2.5-7, 2.5-9, 2.5-10, 3-4, 3-5, 3-6, 3-7, 3-8, 3-9, 3-10, 5-10, 5-20, 5-30, 5-40, 5-50,5-60, 5-70, 5-80, 5-90, 5-100, 7.5-10, 7.5-20, 7.5-30, 7.5-40, 7.5-50, 7.5-60, 7.5-70, 7.5-80, 7.5-90, 7.5-100, 10-10, 10-20, 10-30, 10-40, 10-50, 10-60, 10-70, 10-80, 10-90, 10-100, 10-150, 10-200, 20-30, 20-40, 20-50, 20-60, 20-70, 20-80, 20-90, 20-100, 20-150, 20-200, 30-40, 30-50, 30-60, 30-70, 30-80, 30-90, 30-100, 30-150, 30-200, 40-50, 40-60, 40-70, 40-80, 40-90, 40-100, 40-150, 40-200, 40-300, 50-60, 50-70, 50-80, 50-90, 50-100, 50-150, 50-200, 50-250, 50-300, 60-80, 60-100, 60-150, 60-200, 70-100, 70-150, 70-200, 70-250, 70-300, 80-100, 80-150, 80-200, 80-250, 80-300, 90-100, 90-150, 90-200, 90-250, 90-300, 90-350, 90-400, 100-150, 100-200, 100-250, 100-300, 100-350, or 100-400 mg/kg of the body weight. In some embodiments, the method described herein comprises administering the inhibitor of PD-L1 at a dose of about 0.1, 0.2, 0.3, 0.4, 0.5, 0.6, 0.7, 0.8, 0.9, 1, 1.25, 1.5, 1.75, 2, 2.25, 2.5, 2.75, 3, 3.25, 3.5, 3.75, 4, 4.5, 5, 5.5, 6, 6.5, 7, 7.5, 8, 8.5, 9, 9.5, 10, 12.5, 15, 17.5, 20, 22.5, 25, 27.5, 30, 40, 50, 60, 70, 80, 90 or 100 mg/kg of the body weight.

In some embodiments, the PD-L1 inhibitor (e.g., atezolizumab) is administered at an amount of about 1-10, 1-20, 1-30, 1-40, 1-50, 1-60, 1-70, 1-80, 1-90, 1-100, 1-150, 1-200, 1-250, 1-300, 1-500, 2.5-3, 2.5-4, 2.5-5, 2.5-6, 2.5-7, 2.5-8, 2.5-9, 2.5-10, 2.5-20, 2.5-30, 2.5-40, 2.5-50, 2.5-60, 2.5-70, 2.5-80, 2.5-90, 2.5-100, 2.5-200, 2.5-250, 2.5-300, 2.5-500, 3-10, 3-20, 3-30, 3-40, 3-50, 3-60, 3-70, 3-80, 3-90, 3-100, 3-200, 3-250, 3-300, 3-500, 5-10, 5-10, 5-20, 5-30, 5-40, 5-50,5-60, 5-70, 5-80, 5-90, 5-100, 7.5-10, 7.5-20, 7.5-30, 7.5-40, 7.5-50, 7.5-60, 7.5-70, 7.5-80, 7.5-90, 7.5-100, 10-10, 10-20, 10-30, 10-40, 10-50, 10-60, 10-70, 10-80, 10-90, 10-100, 10-150, 10-200, 20-30, 20-40, 20-50, 20-60, 20-70, 20-80, 20-90, 20-100, 20-150, 20-200, 30-40, 30-50, 30-60, 30-70, 30-80, 30-90, 30-100, 30-150, 30-200, 40-50, 40-60, 40-70, 40-80, 40-90, 40-100, 40-150, 40-200, 40-300, 50-60, 50-70, 50-80, 50-90, 50-100, 50-150, 50-200, 50-250, 50-300, 60-80, 60-100, 60-150, 60-200, 70-100, 70-150, 70-200, 70-250, 70-300, 70-500, 70-750, 70-1000, 70-1500, 70-2000, 70-3000, 80-100, 80-150, 80-200, 80-250, 80-300, 80-500, 80-750, 80-1000, 80-1500, 80-2000, 80-3000, 90-100, 90-150, 90-200, 90-250, 90-300, 90-350, 90-400, 90-500, 90-750, 90-1000, 90-1500, 90-2000, 90-3000, 100-150, 100-200, 100-250, 100-300, 100-350, 100-400, 100-500, 100-600, 100-700, 100-800, 100-900, 100-1000, 100-1500, 100-2000, 100-2500, 100-3000, 100-3500, 100-4000, 200-500, 200-700, 200-1000, 200-1500, 200-2000, 200-2500, 200-3000, 200-3500, 200-4000, 500-1000, 500-1500, 500-2000, 500-2500, 500-3000, 500-3500, or 500-4000 mg per dose. In some embodiments, the PD-L1 inhibitor is administered at an amount of about 500-1500, 600-1500, 700-1500, 800-1500, 900-1500, 1000-1500, or 1100-1300 mg per dose. In some embodiments, the PD-L1 inhibitor is administered at an amount of about 1200 mg per dose.

In some embodiments, the method described herein comprises administering the inhibitor of CTLA-4 (e.g., ipilimumab) at a dose in the range of about 0.5-1, 0.5-2, 0.5-3, 0.5-4, 0.5-5, 0.5-6, 0.5-7, 0.5-8, 0.5-9, 0.5-10, 1-2, 1-3, 1-4, 1-5, 1-6, 1-7, 1-8, 1-9, 1-10, 1-20, 1-30, 1-40, 1-50, 1-60, 1-70, 1-80, 1-90, 1-100, 2-3, 2-4, 2-5, 2-6, 2-7, 2-8, 2-9, 2-10, 2-20, 2-30, 2-40, 2-50, 2-60, 2-70, 2-80, 2-90, 2-100, 2.5-3, 2.5-3.5, 2.5-4, 2.5-5, 2.5-6, 2.5-7, 2.5-9, 2.5-10, 3-4, 3-5, 3-6, 3-7, 3-8, 3-9, 3-10, 5-10, 5-20, 5-30, 5-40, 5-50,5-60, 5-70, 5-80, 5-90, 5-100, 7.5-10, 7.5-20, 7.5-30, 7.5-40, 7.5-50, 7.5-60, 7.5-70, 7.5-80, 7.5-90, 7.5-100, 10-10, 10-20, 10-30, 10-40, 10-50, 10-60, 10-70, 10-80, 10-90, 10-100, 10-150, 10-200, 20-30, 20-40, 20-50, 20-60, 20-70, 20-80, 20-90, 20-100, 20-150, 20-200, 30-40, 30-50, 30-60, 30-70, 30-80, 30-90, 30-100, 30-150, 30-200, 40-50, 40-60, 40-70, 40-80, 40-90, 40-100, 40-150, 40-200, 40-300, 50-60, 50-70, 50-80, 50-90, 50-100, 50-150, 50-200, 50-250, 50-300 mg/kg of the body weight. In some embodiments, the method described herein comprises administering the inhibitor of CTLA-4 at a dose of about 0.1, 0.2, 0.3, 0.4, 0.5, 0.6, 0.7, 0.8, 0.9, 1, 1.25, 1.5, 1.75, 2, 2.25, 2.5, 2.75, 3, 3.25, 3.5, 3.75, 4, 4.5, 5, 5.5, 6, 6.5, 7, 7.5, 8, 8.5, 9, 9.5, 10, 12.5, 15, 17.5, 20, 22.5, 25, 27.5, 30, 40, 50, 60, 70, 80, 90 or 100 mg/kg of the body weight. In some embodiments, the inhibitor of CTLA-4 is administered at a dose of about 3 mg/kg. In some embodiments, the inhibitor of CTLA-4 is administered at a dose of lower than 3 mg/kg. In some embodiments, the inhibitor of CTLA-4 is administered at a dose of about 0.5, 1, 1.5, 2, 2.5, 3, 3.5, or 4 mg/kg. In some embodiments, the inhibitor of CTLA-4 (e.g., ipilimumab) is administered at a dose of about 3 mg/kg.

In some embodiments, the CTLA-4 inhibitor is administered at an amount of about 1-10, 1-20, 1-30, 1-40, 1-50, 1-60, 1-70, 1-80, 1-90, 1-100, 1-150, 1-200, 1-250, 1-300, 1-500, 2.5-3, 2.5-4, 2.5-5, 2.5-6, 2.5-7, 2.5-8, 2.5-9, 2.5-10, 2.5-20, 2.5-30, 2.5-40, 2.5-50, 2.5-60, 2.5-70, 2.5-80, 2.5-90, 2.5-100, 2.5-200, 2.5-250, 2.5-300, 2.5-500, 3-10, 3-20, 3-30, 3-40, 3-50, 3-60, 3-70, 3-80, 3-90, 3-100, 3-200, 3-250, 3-300, 3-500, 5-10, 5-10, 5-20, 5-30, 5-40, 5-50,5-60, 5-70, 5-80, 5-90, 5-100, 7.5-10, 7.5-20, 7.5-30, 7.5-40, 7.5-50, 7.5-60, 7.5-70, 7.5-80, 7.5-90, 7.5-100, 10-10, 10-20, 10-30, 10-40, 10-50, 10-60, 10-70, 10-80, 10-90, 10-100, 10-150, 10-200, 20-30, 20-40, 20-50, 20-60, 20-70, 20-80, 20-90, 20-100, 20-150, 20-200, 30-40, 30-50, 30-60, 30-70, 30-80, 30-90, 30-100, 30-150, 30-200, 40-50, 40-60, 40-70, 40-80, 40-90, 40-100, 40-150, 40-200, 40-300, 50-60, 50-70, 50-80, 50-90, 50-100, 50-150, 50-200, 50-250, 50-300, 60-80, 60-100, 60-150, 60-200, 70-100, 70-150, 70-200, 70-250, 70-300, 70-500, 70-750, 70-1000, 70-1500, 70-2000, 70-3000, 80-100, 80-150, 80-200, 80-250, 80-300, 80-500, 80-750, 80-1000, 80-1500, 80-2000, 80-3000, 90-100, 90-150, 90-200, 90-250, 90-300, 90-350, 90-400, 90-500, 90-750, 90-1000, 90-1500, 90-2000, 90-3000, 100-150, 100-200, 100-250, 100-300, 100-350, 100-400, 100-500, 100-600, 100-700, 100-800, 100-900, 100-1000, 100-1500, 100-2000, 100-2500, 100-3000, 100-3500, 100-4000, 200-500, 200-700, 200-1000, 200-1500, 200-2000, 200-2500, 200-3000, 200-3500, 200-4000, 500-1000, 500-1500, 500-2000, 500-2500, 500-3000, 500-3500, or 500-4000 mg per dose. In some embodiments, the CTLA-4 inhibitor is administered at an amount of about 10-30, 10-50, 10-80, 10-100, 10-125, 10-150, 10-175, 10-200, 10-250, 10-300, 10-400, 20-50, 20-100, 20-125, 20-150, 20-175, 20-200, 20-250, 20-300, 20-400, 30-50, 30-80, 30-100, 30-125, 30-150, 30-175, 30-200, 30-250, 30-300, 30-400, 40-50, 40-80, 40-100, 40-125, 40-150, 40-175, 40-200, 40-250, 40-300, 40-400, 50-80, 50-100, 50-125, 50-150, 50-175, 50-200, 50-250, 50-300, or 50-400 mg per dose.

In some embodiments, the use of plinabulin can reduce the incidence of Grade 3/4 immunotherapy related adverse event by at least about 1%, 2%, 3%, 4%, 5%, 10%, 12.5%, 15%, 17.5%, 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 95%, or 100%. In some embodiments, the use of plinabulin can reduce the incidence of Grade 3/4 immunotherapy related adverse event by at least about 5%, 10%, 12.5%, 15%, 17.5%, 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 95%, or 100%. In some embodiments, the use of plinabulin can reduce the incidence of Grade 3/4 immunotherapy related adverse event by less than about 5%, 10%, 12.5%, 15%, 17.5%, 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 95%, or 100%. In some embodiments, the use of plinabulin can reduce the incidence of Grade 3/4 immunotherapy related adverse event in the range of about 1% - 5%, 1%-10%, 1%-15%, 1% - 20%, 1% - 30%, 1% - 40%, 1%-50%, 2.5%-10%, 2.5%-15%, 2.5% - 20%, 2.5% - 30%, 5%-10%, 5%-15%, 5% - 20%, 5% - 30%,5% - 40%, 10%-40%, 12.5%-40%, 5% - 50%, 10%-50%, 12.5%-50%, 15%-50%, 17.5%-50%, 20%-50%, 25%-50%, 27.5%-50%, 30%-50%, 5% - 60%, 10%-60%, 12.5%-60%, 15%-60%, 17.5%-60%, 20%-60%, 25%-60%, 27.5%-60%, 30%-60%, 35%-60%, 37.5%-60%, 40%-60%, 45%-70%, or 50%-80%.

In some embodiments, the use of plinabulin can reduce the duration of of Grade 3/4 immunotherapy related adverse event by about 1%, 2%, 3%, 4%, 5%, 10%, 12.5%, 15%, 17.5%, 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 95%, 100%, 110%, 120%, 130%, 140%, 150%, 160%, 170%, 180%, 190%, 200%, 225%, 250%, 275%, 300%, 350% 400%, 450%, 500%, 600%, 700%, 800%, 900%, 10 times, 11 times, 12 times, 13 times, 14 times, 15 times, or 16 times. In some embodiments, the use of plinabulin can reduce the duration of Grade 3/4 immunotherapy related adverse event by greater than about 1%, 2%, 3%, 4%, 5%, 10%, 12.5%, 15%, 17.5%, 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 95%, 100%, 110%, 120%, 130%, 140%, 150%, 160%, 170%, 180%, 190%, 200%, 225%, 250%, 275%, 300%, 350% 400%, 450%, 500%, 600%, 700%, 800%, 900%, 10 times, 11 times, 12 times, 13 times, 14 times, 15 times, or 16 times. In some embodiments, the use of plinabulin can reduce the duration of Grade 3/4 immunotherapy related adverse event by less than about 5%, 10%, 12.5%, 15%, 17.5%, 20%, 22.5%, 25%, 27.5%, 30%, 32.5%, 35%, 37.5%, 40%, 42.5%, 45%, 47.5%, 50%, 52.5%, 55%, 57.5%, 60%, 62.5%, 65%, 67.5%, 70%, 72.5%, 75%, 77.5%, 80%, 82.5%, 85%, 87.5%, 90%, 95%, 100%, 110%, 120%, 130%, 140%, 150%, 160%, 170%, 180%, 190%, 200%, 225%, 250%, 275%, 300%, 350% 400%, 450%, 500%, 600%, 700%, 800%, 900%, 10 times, 11 times, 12 times, 13 times, 14 times, 15 times, or 16 times. In some embodiments, the use of plinabulin can reduce the duration of Grade 3/4 immunotherapy related adverse event in the range of about 5%-10%, 5%-20%, 5% - 30%, 5% - 40%, 5% - 50%,5% - 60%, 5% - 70%, 5% - 80%, 5% - 100%, 5% - 2 times, 5% - 5 times, 5% -15 times, 20% - 10 times, or 50%-500%.

Some embodiments relate to a method of treating or preventing an inflammatory skin or joint condition in a subject, wherein the method comprises topically administering an effective amount of plinabulin to the subject in need thereof.

In some embodiments, the skin or joint disorder is psoriasis. In some embodiments, the skin or joint disorder is arthritis. In some embodiments, the skin or joint disorder is rheumatoid arthritis. In some embodiments, the skin or joint disorder is selected from ankylosing spondylitis, psoriatic arthritis, sarcoidosis, systemic lupus erythematosus, or atopic dermatitis.

Some embodiments relate to a method of treating or preventing an inflammatory disease in a subject, comprising administering an effective amount of plinabulin to the subject in need thereof, wherein the inflammatory disease is selected from the group consisting of chronic articular rheumatism, chronic obstructive pulmonary disease, asthma, ankylosing spondylitis, psoriatic arthritis, sarcoidosis, systemic lupus erythematosus, inflammatory bowel disease, atopic dermatitis, and multiple sclerosis.

In some embodiments, the plinabulin is administered parenterally. In some embodiments, the plinabulin is administered orally. In some embodiments, the plinabulin is administered topically.

Some embodiments relate to a method of treating or preventing chronic obstructive pulmonary disease or asthma in a subject comprising administering an effective amount of plinabulin to the subject in need thereof through an inhaler.

### Composition and Administration

The methods described herein can include administering a composition comprising plinabulin. In some embodiments, the composition described herein can further include one or more pharmaceutically acceptable diluents. In some embodiments, the pharmaceutically acceptable diluent can include Kolliphor^{®} HS 15 (Polyethylene glycol (15)-hydroxystearate). In some embodiments, the pharmaceutically acceptable diluent can include Kolliphor^{®} EL, Kolliphor^{®} RH40, Kolliphor^{®} P 188, Kolliphor^{®} P 407, and Kolliphor^{®} F-68. In some embodiments, the pharmaceutically acceptable diluent can include propylene glycol. In some embodiments, the pharmaceutically acceptable diluents can include kolliphor and propylene glycol. In some embodiments, the pharmaceutically acceptable diluents can include kolliphor and propylene glycol, wherein the kolliphor is about 40% by weight and propylene glycol is about 60% by weight based on the total weight of the diluents. In some embodiments, the composition can further include one or more other pharmaceutically acceptable excipients.

Some embodiments relate to a topical formulation, comprising plinabulin at a concentration effective to inhibit PDE4 activity without reducing vascular proliferation or density.

The amount of plinabulin in the topical formulation, when applied to the skin, nail, hair, hand or foot, is sufficient to produces a desired pharmacological result either locally at the place of application or systemically as a result of transdermal passage of an active ingredient in the material.

Some embodiments relate to a topical formulation, comprising plinabulin at a concentration in the range of about 0.1% to about 10% by weight of the total formulation. In some embodiments, plinabulin is at a concentration in the range of about 0.05%-0.1%,0.05%-0.2%, 0.05%-0.3, 0.05%-0.4%, 0.05%-0.5%, 0.05%-1%, 0.05%-2%, 0.05%-3%, 0.05%-4%, 0.05%-5%, 0.05%-6%, 0.05%-7%, 0.05%-8%, 0.05%-9%, 0.05%-10%, 0.1%-0.2%, 0.1%-0.3, 0.1%-0.4%, 0.1%-0.5%, 0.1%-1%, 0.1%-2%, 0.1%-3%, 0.1%-4%, 0.1%-5%, 0.1%-6%, 0.1%-7%, 0.1%-8%, 0.1%-9%, 0.1%-10%, 0.5%-1%, 0.5%-2%, 0.5%-3%, 0.5%-4%, 0.5%-5%, 0.5%-6%, 0.5%-7%, 0.5%-8%, 0.5%-9%, 0.1%-0.2%, 1%-2%, 1%-3%, 1%-4%, 1%-5%, 1%-6%, 1%-7%, 1%-8%, 1%-9%, 1%-10%, 2%-3%, 2%-4%, 2%-5%, 2%-6%, 2%-7%, 2%-8%, 2%-9%, 2%-10%, 3%-4%, 3%-5%, 3%-6%, 3%-7%, 3%-8%, 3%-9%, 3%-10%, 4%-5%, 4%-6%, 4%-7%, 4%-8%, 4%-9%, 4%-10%, 5%-6%, 5%-7%, 5%-8%, 5%-9%, 5%-10%, 6%-7%, 6%-8%, 6%-9%, 6%-10%, 7%-8%, 7%-9%, 7%-10%, 8%-9%, 9%-10%, 1%-20%, or 5%-20%, by weight of the total formulation. In some embodiments, the concentration of plinabulin is in the range of about 0.5%-2.5%, 1%-3%, or 2%-4% by weight of the total formulation. In some embodiments, plinabulin has a concentration of about 0.5%, 1%, 1.5%, 2%, 2.5%, 3%, 3.5%, 4%, 4.5%, 5%, 6%, 7%, 8%, 9%, or 10% by weight of the total formulation. In some embodiments, plinabulin has a concentration greater than about 0.5%, 1%, 1.5%, 2%, 2.5%, 3%, 3.5%, 4%, 4.5%, 5%, 6%, 7%, 8%, 9%, or 10% by weight of the total formulation. In some embodiments, plinabulin has a concentration less than about 2%, 2.5%, 3%, 3.5%, 4%, 4.5%, 5%, 6%, 7%, 8%, 9%, or 10% by weight of the total formulation. In some embodiments, plinabulin has a concentration of about 0.1%, 0.2%, 0.3%, 0.5%, 1%, 1.5%, 2%, 2.5%, 3%, 3.5%, 4%, 4.5%, 5%, 6%, 7%, 8%, 9%, or 10% by weight of the total formulation.

In some embodiments, the topical formulation includes one or more ingredients selected from white petrolatum, propylene glycol, mono- and di-glycerides, paraffin, butylated hydroxytoluene, or edetate calcium disodium. In some embodiments, the topical formulation includes Polyoxyl 15 hydroxystearate.

The topical formulation can include fluid or semi-solid vehicles that may include but are not limited to polymers, thickeners, buffers, neutralizers, chelating agents, preservatives, surfactants or emulsifiers, antioxidants, waxes or oils, emollients, sunscreens, and a solvent or mixed solvent system. The solvent or mixed solvent system is important to the formation because it is primarily responsible for dissolving the drug. The best solvent or mixed solvent systems are also capable of maintaining clinically relevant levels of the drug in solution despite the addition of a poor solvent to the formulation. The topical compositions useful in the subject invention can be made into a wide variety of product types. These include, but are not limited to, patches, lotions, creams, gels, sticks, sprays, ointments, pastes, foams, mousses, masks, eye ointments, eye or ear drops, impregnated dressings, wipes, cleansers including soaps, body washes and shampoos, and make-up products, such as bases, blushes, lipsticks, and eye shadows, among others. These product types can comprise several types of carrier systems including, but not limited to particles, nanoparticles, and liposomes. The formulation can be selected to maximize delivery to a desired target site in the body. The formulations can also include various conventional colorants, fragrances, thickeners, preservatives, humectants, emollients, demulcents, solubilizing excipients, dispersants, penetration enhancers, plasticizing agents, preservatives, stabilizers, demulsifiers, wetting agents, sunscreens, emulsifiers, moisturizers, astringents, deodorants, and the like, which can be added to provide additional benefits such as, for example, improving the feel and/or appearance of the topical preparation.

In some embodiments, the formulation is in the form of lotion, cream, ointment, gel, emulsion, or suspension.

Lotions, which are preparations that are to be applied to the skin, nail, hair, claw or hoof surface without friction, are typically liquid or semi-liquid preparations in which finely divided solid, waxy, or liquid are dispersed. Lotions will typically contain suspending agents to produce better dispersions as well as compounds useful for localizing and holding the active agent in contact with the skin, nail, hair, claw or hoof, e.g., methylcellulose, sodium carboxymethyl-cellulose, or the like.

Creams containing the active agent for delivery according to the present invention are viscous liquid or semisolid emulsions, either oil-in-water or water-in-oil. Cream bases are water-washable, and contain an oil phase, an emulsifier and an aqueous phase. The oil phase is generally comprised of petrolatum or a fatty alcohol, such as cetyl- or stearyl alcohol; the aqueous phase usually, although not necessarily, exceeds the oil phase in volume, and generally contains a humectant.

Gel formulations can also be used in connection with the present invention. As will be appreciated by those working in the field of topical drug formulation, gels are semisolid. Single-phase gels contain organic macromolecules distributed substantially uniformly throughout the carrier liquid, which is typically aqueous, but also may be a solvent or solvent blend. In various embodiments, conventional gelling agents can be used. In an exemplary embodiment, cellulose or its derivatives are used. In an exemplary embodiment, hydroxypropyl methyl cellulose, such as Methocel E4M, is used. Other gelling agents include methyl cellulose, hydroxypropyl cellulose, hydroxypropyl methyl cellulose, cellulose acetate, ethyl cellulose, methyl hydroxy ethyl cellulose, hydroxy ethyl cellulose, and cellulose gum. Cellulose based gelling agents, particularly hydroxymethylcellulose and hydroxypropyl methyl cellulose, are also useful in some embodiments. In some embodiments, cross-linked acrylic polymers including Carbopol may be used.

In one embodiment, the formulation of the invention is viscous enough to form a firm gel. In one embodiment, the viscosity is in the range of 25,000-300,000 cps (centipoise) or 75,000-200,000 cps, based on Brookfield (LV) analysis.

For ease of preparation, it may be convenient to prepare a first gel composition, named speed-gel herein, which can be used to add to other components in the formulation of a final composition for topical administration. There are several possible formulations of the speed-gel. For example, a speed-gel may be prepared by mixing lecithin organogel (L.O.), as a 1:1 (m/m) mixture of lecithin and isopropyl myristate, with LID oil (a 1:1 [m/m] mixture of L.O. and docusate sodium), dissolving additional docusate sodium powder into this mixture, and then adding aqueous urea.

Ointments, which are semisolid preparations, are typically based on petrolatum or other petroleum derivatives. As will be appreciated by the ordinarily skilled artisan, the specific ointment base to be used is one that provides for optimum delivery for the active agent chosen for a given formulation, and, preferably, provides for other desired characteristics as well, e.g., emolliency or the like. As with other carriers or vehicles, an ointment base should be inert, stable, nonirritating and non-sensitizing. As explained in Remington: The Science and Practice of Pharmacy, 19th Ed. (Easton, Pa.: Mack Publishing Co., 1995), at pages 1399-1404, ointment bases may be grouped in four classes: oleaginous bases; emulsifiable bases; emulsion bases; and water-soluble bases. Oleaginous ointment bases include, for example, vegetable oils, fats obtained from animals, and semisolid hydrocarbons obtained from petroleum. Examples of oleaginous ointment bases include White Ointment USP, Yellow Ointment NF, Oleic Acid USP, Olive Oil USP, Paraffin USP, Petrolatum NF, White Petrolatum USP, Spermaceti Wax USP, Synthetic Spermaceti NF, Starch Glycerite NF, White Wax USP, and Yellow Wax USP. Emulsifiable ointment bases, also known as absorbent ointment bases, contain little or no water and include, for example, hydroxystearin sulfate, anhydrous lanolin and hydrophilic petrolatum. Emulsion ointment bases are either water-in-oil (W/O) emulsions or oil-in-water (O/W) emulsions, and include, for example, cetyl alcohol, glyceryl monostearate, lanolin and stearic acid. Preferred water-soluble ointment bases are prepared from polyethylene glycols of varying molecular weight; again, reference may be had to Remington: The Science and Practice of Pharmacy, supra, for further information.

Useful formulations of the invention also encompass sprays and aerosols. Sprays generally provide the active agent in an aqueous and/or alcoholic solution which can be misted onto the skin, nail, hair, claw or hoof for delivery. Such sprays include those formulated to provide for concentration of the active agent solution at the site of administration following delivery, e.g., the spray solution can be primarily composed of alcohol or other like volatile liquid in which the drug or active agent can be dissolved. Upon delivery to the skin, nail, hair, claw or hoof, the carrier evaporates, leaving concentrated active agent at the site of administration. Examples of aerosol technology are disclosed in U.S. Pat. Nos. 6,682,716; 6,716,415; 6,716,417; 6,783,753; 7,029,658; and 7,033,575.

The topical pharmaceutical compositions may also comprise suitable solid or gel phase carriers. Examples of such carriers include but are not limited to calcium carbonate, calcium phosphate, various sugars, starches, cellulose derivatives, gelatin, and polymers such as polyethylene glycols.

The topical pharmaceutical compositions may also comprise a suitable emulsifier which refers to an agent that enhances or facilitates mixing and suspending oil-in-water or water-in-oil. The emulsifying agent used herein may consist of a single emulsifying agent or may be a nonionic, anionic, cationic or amphoteric surfactant or blend of two or more such surfactants; preferred for use herein are nonionic or anionic emulsifiers. Such surface-active agents are described in "McCutcheon's Detergent and Emulsifiers," North American Edition, 1980 Annual published by the McCutcheon Division, MC Publishing Company, 175 Rock Road, Glen Rock, N.J. 07452, USA.

Examples of useful ionic surfactants include sodium caproate, sodium caprylate, sodium caprate, sodium laurate, sodium myristate, sodium myristolate, sodium palmitate, sodium palmitoleate, sodium oleate, sodium ricinoleate, sodium linoleate, sodium linolenate, sodium stearate, sodium lauryl sulfate (dodecyl), sodium tetradecyl sulfate, sodium lauryl sarcosinate, sodium dioctyl sulfosuccinate, sodium cholate, sodium taurocholate, sodium glycocholate, sodium deoxycholate, sodium taurodeoxycholate, sodium glycodeoxycholate, sodium ursodeoxycholate, sodium chenodeoxycholate, sodium taurochenodeoxycholate, sodium glyco cheno deoxycholate, sodium cholylsarcosinate, sodium N-methyl taurocholate, egg yolk phosphatides, hydrogenated soy lecithin, dimyristoyl lecithin, lecithin, hydroxylated lecithin, lysophosphatidylcholine, cardiolipin, sphingomyelin, phosphatidylcholine, phosphatidyl ethanolamine, phosphatidic acid, phosphatidyl glycerol, phosphatidyl serine, diethanolamine, phospholipids, polyoxyethylene-10 oleyl ether phosphate, esterification products of fatty alcohols or fatty alcohol ethoxylates, with phosphoric acid or anhydride, ether carboxylates (by oxidation of terminal OH group of, fatty alcohol ethoxylates), succinylated monoglycerides, sodium stearyl fumarate, stearoyl propylene glycol hydrogen succinate, mono/diacetylated tartaric acid esters of mono- and diglycerides, citric acid esters of mono-, diglycerides, glyceryl-lacto esters of fatty acids, acyl lactylates, lactylic esters of fatty acids, sodium stearoyl-2-lactylate, sodium stearoyl lactylate, alginate salts, propylene glycol alginate, ethoxylated alkyl sulfates, alkyl benzene sulfones, alpha.-olefin sulfonates, acyl isethionates, acyl taurates, alkyl glyceryl ether sulfonates, sodium octyl sulfosuccinate, sodium undecylenamideo-MEA-sulfosuccinate, hexadecyl triammonium bromide, decyl trimethyl ammonium bromide, cetyl trimethyl ammonium bromide, dodecyl ammonium chloride, alkyl benzyldimethylammonium salts, diisobutyl phenoxyethoxydimethyl benzylammonium salts, alkylpyridinium salts, betaines (trialkylglycine), lauryl betaine (N-lauryl,N,N-dimethylglycine), and ethoxylated amines (polyoxyethylene-15 coconut amine). For simplicity, typical counterions are provided above. It will be appreciated by one skilled in the art, however, that any bioacceptable counterion may be used. For example, although the fatty acids are shown as sodium salts, other cation counterions can also be used, such as, for example, alkali metal cations or ammonium. Formulations of the invention may include one or more of the ionic surfactants above.

Some high molecular weight alcohols include such as cetearyl alcohol, cetyl alcohol, stearyl alcohol, emulsifying wax, glyceryl monostearate, and oleyl alcohol. Other examples are ethylene glycol distearate, sorbitan tristearate, propylene glycol monostearate, sorbitan monooleate, sorbitan monostearate (SPAN 60), diethylene glycol monolaurate, sorbitan monopalmitate, sucrose dioleate, sucrose stearate (CRODESTA F-160), polyoxyethylene lauryl ether (BRIJ 30), polyoxyethylene (2) stearyl ether (BRU 72), polyoxyethylene (21) stearyl ether (BRIJ 721), polyoxyethylene monostearate (Myrj 45), polyoxyethylene (20) sorbitan monolaurate (TWEEN 20, polysorbate 20), polyoxyethylene (20) sorbitan monopalmitate (TWEEN 40, polysorbate 40), polyoxyethylene (20) sorbitan monostearate (TWEEN 60, polysorbate 60), polyoxyethylene (20) sorbitan monooleate (TWEEN 80, polysorbate 80), other non-ionic polyoxyalkylene derivatives of hexitol anhydride partial long chain fatty acid esters, and sodium oleate. In an exemplary embodiment, the emulsifier is octyldodecanol. In an exemplary embodiment, xanthan gum or a xanthan gum blend is used. Cholesterol and cholesterol derivatives may also be employed in externally used emulsions and promote w/o emulsions.

Some suitable nonionic emulsifying agents are those with hydrophilelipophile balances (HLB) of about 3 to 6 for w/o system and 8 to 18 for o/w system as determined by the method described by Paul L. Lindner in "Emulsions and Emulsion", edited by Kenneth Lissant, published by Dekker, New York, N.Y., 1974, pages 188-190. More preferred for use herein are one or more nonionic surfactants that produce a system having HLB of about 8 to about 18.

Examples of such nonionic emulsifiers include but are not limited to "BRIJ 72", the trade name for a polyoxyethylene (2) stearyl ether having an HLB of 4.9; "BRIJ 721", the trade name for a polyoxyethylene (21) stearyl ether having an HLB of 15.5, "Brij 30", the trade name for polyoxyethylene lauryl ether having an HLB of 9.7; "Polawax", the trade name for emulsifying wax having an HLB of 8.0; "Span 60", the trade name for sorbitan monostearate having an HLB of 4.7; "Crodesta F-160", the trade name for sucrose stearate" having an HLB of 14.5. All of these materials are available from Ruger Chemicals Inc.; Croda; ICI Americas, Inc.; Spectrum Chemicals; and BASF. When the topical formulations of the present invention contain at least one emulsifying agent, each emulsifying agent is present in amount from about 0.5 to about 2.5 wt %, preferably 0.5 to 2.0%, more preferably 1.0% or 1.8%. Preferably the emulsifying agent comprises a mixture of steareth 21 (at about 1.8%) and steareth 2 (at about 1.0%).

The topical pharmaceutical compositions may also comprise suitable emollients. Emollients are materials used for the prevention or relief of dryness, as well as for the protection of the skin, nail, hair, claw or hoof. Useful emollients include, but are not limited to, hydrocarbon oils, waxes, silicone, cetyl alcohol, isopropyl myristate, stearyl alcohol, oleyl alcohol, octyl hydroxystearate, glycerin, other fatty alcohols including short or medium chain fatty alcohols having a carbon length of up to 18, medium or short chain fatty acid triglycerides, esters such as fatty acid esters, lecithins and related polar compounds such as phosphatidylcholine, phosphatidylethanolamine, phosphatidylserine, phosphatidylinositol, phosphatidic acid, lyso-phosphatidylcholine, lyso-phosphatidylethanolamine, and sphingomyelin and the like. Other suitable emollients include triglyceride oils like vegetable oils such as wheat germ, maize, sunflower, karite, castor, sweet almond, macadamia, apricot, soybean, cottonseed, alfalfa, poppy, pumpkinseed, sesame, cucumber, rapeseed, avocado, hazelnut, grape seed, blackcurrant seed, evening primrose, millet, barley, quinoa, olive, rye, safflower, candlenut, soya, palm, passion flower, or musk rose oil; triglycerides of caprylic/capric acid, such as those sold under the tradenames MIGLYOL^{®} (Condea Chemie, Germany) and CRODAMOL (Croda, Inc., Edison, N.J.); fatty alcohols such as caprylic alcohol, lauryl alcohol, myristyl alcohol, cetyl alcohol, and stearyl alcohol; and fatty esters such as oleyl acetate, isotridecyl benzoate, diisooctyl sebacate, isopropyl myristate, cetyl octanoate, isopropyl palmitate, butyl stearate, hexyl laurate, myristyl myristate, decyl oleate, hexyldecyl dimethyloctanoate, cetyl lactate, myristyl lactate, lanoline acetate, isocetyl stearate, isocetyl isostearate, cholesteryl 12-hydroxystearate, dipentaerythritol fatty acid ester, and isostearyl malate. A wide variety of suitable emollients are known and can be used herein. See e.g., Sagarin, Cosmetics, Science and Technology, 2nd Edition, Vol. 1, pp. 32-43 (1972), and U.S. Pat. No. 4,919,934, to Deckner et al., issued Apr. 24, 1990, both of which are incorporated herein by reference in their entirety. These materials are available from Ruger Chemical Co, (Irvington, N.J.).

When the topical formulations of the present invention contain at least one emollient, each emollient is present in an amount from about 0.1 to 15%, preferably 0.1 to about 3.0, more preferably 0.5, 1.0, or 2.5 wt %. Preferably the emollient is a mixture of cetyl alcohol, isopropyl myristate and stearyl alcohol in a 1/5/2 ratio. The emollient may also be a mixture of cetyl alcohol and stearyl alcohol in a 1/2 ratio.

The topical pharmaceutical compositions may also comprise suitable antioxidants, substances known to inhibit oxidation. Antioxidants suitable for use in accordance with the present invention include, but are not limited to, butylated hydroxytoluene, ascorbic acid, sodium ascorbate, calcium ascorbate, ascorbic palmitate, butylated hydroxyanisole, 2,4,5-trihydroxybutyrophenone, 4-hydroxymethyl-2,6-di-tertbutylphenol, erythorbic acid, gum guaiac, propyl gallate, thiodipropionic acid, dilauryl thiodipropionate, tert-butylhydroquinone and tocopherols such as vitamin E, and the like, including pharmaceutically acceptable salts and esters of these compounds. Preferably, the antioxidant is butylated hydroxytoluene, butylated hydroxyanisole, propyl gallate, ascorbic acid, pharmaceutically acceptable salts or esters thereof, or mixtures thereof. Most preferably, the antioxidant is butylated hydroxytoluene. These materials are available from Ruger Chemical Co, (Irvington, N.J.). Antioxidants that may be incorporated into the formulations of the invention include natural antioxidants prepared from plant extracts, such as extracts from aloe vera; avocado; chamomile; echinacea; ginko biloba; ginseng; green tea; heather; jojoba; lavender; lemon grass; licorice; mallow; oats; peppermint; St. John's wort; willow; wintergreen; wheat wild yam extract; marine extracts; and mixtures thereof.

When the topical formulations of the present invention contain at least one antioxidant, the total amount of antioxidant present is from about 0.001 to 0.5 wt %, preferably 0.05 to about 0.5 wt %, more preferably 0.1%.

The topical pharmaceutical compositions may also comprise suitable preservatives. Preservatives are compounds added to a pharmaceutical formulation to act as an anti-microbial agent. Among preservatives known in the art as being effective and acceptable in parenteral formulations are benzalkonium chloride, benzethonium, chlorohexidine, phenol, m-cresol, benzyl alcohol, methylparaben, propylparaben and other parabens, chlorobutanol, o-cresol, p-cresol, chlorocresol, phenylmercuric nitrate, thimerosal, benzoic acid, and various mixtures thereof. See, e.g., Wallhausser, K.-H., Develop. Biol. Standard, 24:9-28 (1974) (S. Krager, Basel). Preferably, the preservative is selected from methylparaben, propylparaben and mixtures thereof. These materials are available from Inolex Chemical Co (Philadelphia, Pa.) or Spectrum Chemicals.

When the topical formulations of the present invention contain at least one preservative, the total amount of preservative present is from about 0.01 to about 0.5 wt %, preferably from about 0.1 to 0.5%, more preferably from about 0.03 to about 0.15. Preferably the preservative is a mixture of methylparaben and proplybarben in a 5/1 ratio. When alcohol is used as a preservative, the amount is usually 15 to 20%.

The topical pharmaceutical compositions may also comprise suitable chelating agents to form complexes with metal cations that do not cross a lipid bilayer. Examples of suitable chelating agents include ethylene diamine tetraacetic acid (EDTA), ethylene glycol-bis(beta-aminoethyl ether)-N,N,N',N'-tetraacetic acid (EGTA) and 8-Amino-2-[(2-amino-5-methylphenoxy)methyl]-6-methoxyquinoline-N,N,N',N'-tetraacetic acid, tetrapotassium salt (QUIN-2). Preferably the chelating agents are EDTA and citric acid. A chelating agent may comprise salts of the above, such as edetate disodium, for example. These materials are available from Spectrum Chemicals.

When the topical formulations of the present invention contain at least one chelating agent, the total amount of chelating agent present is from about 0.005% to 2.0% by weight, preferably from about 0.05% to about 0.5 wt %, more preferably about 0.1% by weight.

The topical pharmaceutical compositions may also comprise suitable neutralizing agents used to adjust the pH of the formulation to within a pharmaceutically acceptable range. Examples of neutralizing agents include but are not limited to trolamine, tromethamine, sodium hydroxide, hydrochloric acid, sodium carbonate, citric acid, acetic acid and corresponding acids or bases thereof. Such materials are available from are available from Spectrum Chemicals (Gardena, Calif.).

When the topical formulations of the present invention contain at least one neutralizing agent, the total amount of neutralizing agent present is from about 0.1 wt to about 10 wt %, preferably 0.1 wt % to about 5.0 wt %, and more preferably about 1.0 wt %. The neutralizing agent is generally added in whatever amount is required to bring the formulation to the desired pH. In one embodiment, the pH is about 6.0 to about 8.0. In one embodiment, the pH is about 3.0 to about 4.0.

The topical pharmaceutical compositions may also comprise suitable thickening or viscosity increasing agents. These components are diffusible compounds capable of increasing the viscosity of a polymer-containing solution through the interaction of the agent with the polymer. For example, CARBOPOL ULTREZ 10, polymethyl methacrylate (PMMA), and fumed silica may be used as a viscosityincreasing agent. These materials are available from Noveon Chemicals, Cleveland, Ohio. Other examples of thickeners include monoglycerides and fatty alcohols, fatty acid esters of alcohols having from about 3 to about 16 carbon atoms. Examples of suitable monoglycerides are glyceryl monostearate and glyceryl monopalmitate. Examples of fatty alcohols are cetyl alcohol and stearyl alcohol. Examples of suitable esters are myristyl stearate and cetyl stearate. The monoglyceride also functions as an auxiliary emulsifier. Other emollients or oleaginous material which may be employed include petrolatum, glyceryl monooleate, myristyl alcohol, and isopropyl palmitate. In one embodiment, the thickener is used in combination with an emulsifying agent.

When the topical formulations of the present invention contain at least one viscosity increasing agent, the total amount of viscosity increasing agent present is from about 0.25% to about 5.0% by weight, preferably from about 0.25% to about 1.0 wt %, and more preferably from about 0.4% to about 0.6% by weight.

The topical pharmaceutical compositions may also comprise a disintegrating agent including starch, e.g., a natural starch such as corn starch or potato starch, a pregelatinized starch such as National 1551 or Amijele^{®}, or sodium starch glycolate such as Promogel^{®} or Explotab^{®}; a cellulose such as a wood product, microcrystalline cellulose, e.g., Avicel^{®}, Avicel^{®} PH101, Avicel^{®} PH102, Avicel^{®} PH105, Elcema^{®} P100, Emcocel^{®}, Vivacel^{®}, Ming Tia^{®}, and Solka-Floc^{®}, methylcellulose, croscarmellose, or a cross-linked cellulose, such as cross-linked sodium carboxymethylcellulose (Ac-Di-Sol^{®}), cross-linked carboxymethylcellulose, or cross-linked croscarmellose; a cross-linked starch such as sodium starch glycolate; a cross-linked polymer such as crosspovidone; a cross-linked polyvinylpyrrolidone; alginate such as alginic acid or a salt of alginic acid such as sodium alginate; a clay such as Veegum^{®} HV (magnesium aluminum silicate); a gum such as agar, guar, locust bean, Karaya, pectin, or tragacanth; sodium starch glycolate; bentonite; a natural sponge; a surfactant; a resin such as a cation-exchange resin; citrus pulp; sodium lauryl sulfate; sodium lauryl sulfate in combination starch; and the like.

The topical pharmaceutical compositions may also comprise suitable nail penetration enhancers. Examples of nail penetration enhancers include mercaptan compounds, sulfites and bisulfites, keratolytic agents and surfactants. Nail penetration enhancers suitable for use in the invention are described in greater detail in Malhotra et al., J. Pharm. Sci., 91:2, 312-

The topical pharmaceutical compositions may also comprise an anti-foaming anti-whitening agent that increases the elegancy of the cream or lotion and inhibits the formation of a white soapy look upon rubbing the cream or lotion on the skin. An example of such material includes silicone fluid. Other anti-foaming agents include simethicone, polyglycol, and sorbitan sesquioleate.

The topical pharmaceutical compositions may also comprise a post-foaming agent. "Post-foaming" refers to a gel that remains a gel as it is expelled from a container but foams up after it is spread over the skin. Post-foaming agents include saturated aliphatic hydrocarbons having from 4-6 carbon atoms, such as butane, pentane and hexane (in particular is opentane and isobutene). Other suitable post-foaming agents include partially, or wholly halogenated hydrocarbons, such as trichlorofluoroethane. Also, mixtures of aliphatic and halogenated hydrocarbon propellants, or post-foaming agents can be used. Generally suitable post-foaming agents are those substances that have a low solubility in water, for example less than about 20 cc of gas in 100 grams of water at one atmosphere and 20.°C.

The topical pharmaceutical compositions may also comprise one or more suitable solvents. The ability of any solid substance (solute) to dissolve in any liquid substance (solvent) is dependent upon the physical properties of the solute and the solvent. When solutes and solvents have similar physical properties the solubility of the solute in the solvent will be the greatest. This gives rise to the traditional understanding that "like dissolves like." Solvents can be characterized in one extreme as non-polar, lipophilic oils, while in the other extreme as polar hydrophilic solvents. Oily solvents dissolve other non-polar substances by Van der Waals interactions while water and other hydrophilic solvents dissolve polar substances by ionic, dipole, or hydrogen bonding interactions. All solvents can be listed along a continuum from the least polar, i.e. hydrocarbons such as decane, to the most polar solvent being water. A solute will have its greatest solubility in solvents having equivalent polarity. Thus, for drugs having minimal solubility in water, less polar solvents will provide improved solubility with the solvent having polarity nearly equivalent to the solute providing maximum solubility. Most drugs have intermediate polarity, and thus experience maximum solubility in solvents such as propylene glycol or ethanol, which are significantly less polar than water. If the drug has greater solubility in propylene glycol (for example 8% (w/w)) than in water (for example 0.1% (w/w)), then addition of water to propylene glycol should decrease the maximum amount of drug solubility for the solvent mixture compared with pure propylene glycol. Addition of a poor solvent to an excellent solvent will decrease the maximum solubility for the blend compared with the maximum solubility in the excellent solvent.

When compounds are incorporated into topical formulations the concentration of active ingredient in the formulation may be limited by the solubility of the active ingredient in the chosen solvent and/or carrier. Non-lipophilic drugs typically display very low solubility in pharmaceutically acceptable solvents and/or carriers. For example, the solubility of some compounds in the invention in water is less than 0.00025% wt/wt. The solubility of the same compounds in the invention can be less than about 2% wt/wt in either propylene glycol or isopropyl myristate.

Examples of solubilizing excipients include polyethoxylated fatty acids, PEG-fatty acid diesters, PEG-fatty acid mono-ester and di-ester mixtures, polyethylene glycol glycerol fatty acid esters, alcohol-oil transesterification products, polyglycerized fatty acids, propylene glycol fatty acid esters, mixtures of propylene glycol esters-glycerol esters, mono- and diglycerides, sterol and sterol derivatives, polyethylene glycol sorbitan fatty acid esters, polyethylene glycol alkyl ethers, sugar esters, polyethylene glycol alkyl phenols, polyoxyethylene-polyoxypropylene block copolymers, sorbitan fatty acid esters, lower alcohol fatty acid esters, ionic surfactants, tocopherol esters, and sterol esters. In one embodiment of the present invention, ethylhexyl hydroxystearate is the solvent used to dissolve the compounds described herein. In one embodiment of the present invention, diethylene glycol monoethyl ether (DGME) is the solvent used to dissolve the compounds described herein. In one embodiment of the present invention, diethylene glycol monoethyl ether (DGME) is the solvent used to dissolve a compound of the invention. The compounds in the invention useful in the present formulation are believed to have a solubility of from about 10% wt/wt to about 25% wt/wt in DGME. In another embodiment a DGME water cosolvent system is used to dissolve the compounds described herein. In another embodiment a DGME water cosolvent system is used to dissolve a compound of the invention. The solvent capacity of DGME drops when water is added; however, the DGME/water cosolvent system can be designed to maintain the desired concentration of from about 0.1% to about 5% wt/wt active ingredient. Preferably the active ingredient is present from about 0.5% to about 3% wt/wt, and more preferably at about 1% wt/wt, in the as-applied topical formulations. Because DGME is less volatile than water, as the topical formulation evaporates upon application, the active agent becomes more soluble in the cream formulation. This increased solubility reduces the likelihood of reduced bioavailability caused by the drug precipitating on the surface of the skin, nail, hair, claw or hoof.

In one embodiment, the vehicle is lipophilic. Lipophilic materials include oleaginous material such as petrolatum, mineral oil thickened or gelled with polyethylene, high molecular weight paraffin waxes, mono and diglycerides of fatty acids gelled with high molecular weight fatty acids or polyamide complex of hydroxystearate, propylene glycol isostearate or isostearyl alcohol gelled with high molecular weight fatty acids, and mixtures thereof.

Standard pharmaceutical formulation techniques can be used to make the pharmaceutical compositions described herein, such as those disclosed in Remington's The Science and Practice of Pharmacy, 21st Ed., Lippincott Williams & Wilkins (2005), incorporated herein by reference in its entirety. Accordingly, some embodiments include pharmaceutical compositions comprising: (a) a safe and therapeutically effective amount of Plinabulin or pharmaceutically acceptable salts thereof; and (b) a pharmaceutically acceptable carrier, diluent, excipient or combination thereof.

In addition to a topical route of administration, some embodiments include administration via any of the other accepted modes of administration for agents that serve similar utilities including, but not limited to, orally, sublingually, buccally, subcutaneously, intravenously, intranasally, intradermally, intraperitoneally, intramuscularly, intrapulmonarilly, vaginally, rectally, or intraocularly.

The term "pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. In addition, various adjuvants such as are commonly used in the art may be included. Considerations for the inclusion of various components in pharmaceutical compositions are described, e.g., in Gilman et al. (Eds.) (1990); Goodman and Gilman's: The Pharmacological Basis of Therapeutics, 8th Ed., Pergamon Press, which is incorporated herein by reference in its entirety.

Some examples of substances, which can serve as pharmaceutically-acceptable carriers or components thereof, are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as the TWEENS; wetting agents, such sodium lauryl sulfate; coloring agents; flavoring agents; tableting agents, stabilizers; antioxidants; preservatives; pyrogen-free water; isotonic saline; and phosphate buffer solutions.

The compositions described herein are preferably provided in unit dosage form. As used herein, a "unit dosage form" is a composition containing an amount of a compound or composition that is suitable for administration to an animal, preferably a mammalian subject, in a single dose, according to good medical practice. The preparation of a single or unit dosage form however, does not imply that the dosage form is administered once per day or once per course of therapy. Such dosage forms are contemplated to be administered once, twice, thrice or more per day and may be administered as infusion over a period of time (e.g., from about 30 minutes to about 2-6 hours), or administered as a continuous infusion, and may be given more than once during a course of therapy, although a single administration is not specifically excluded. The skilled artisan will recognize that the formulation does not specifically contemplate the entire course of therapy and such decisions are left for those skilled in the art of treatment rather than formulation.

Some embodiments include compositions in any of a variety of suitable forms for a variety of routes for administration other than topical, for example, for oral, sublingual, buccal, nasal, rectal, intradermal, ocular, intracerebral, intracranial, intrathecal, intra-arterial, intravenous, intramuscular, or other parental routes of administration. The skilled artisan will appreciate that oral and nasal compositions include compositions that are administered by inhalation, and made using available methodologies. Depending upon the particular route of administration desired, a variety of pharmaceutically-acceptable carriers well-known in the art may be used. Pharmaceutically-acceptable carriers include, for example, solid or liquid fillers, diluents, hydrotropies, surface-active agents, and encapsulating substances. Optional pharmaceutically-active materials may be included, which do not substantially interfere with the activity of the compound or composition. The amount of carrier employed in conjunction with the compound or composition is sufficient to provide a practical quantity of material for administration per unit dose of the compound. Techniques and compositions for making dosage forms useful in the methods described herein are described in the following references, all incorporated by reference herein: Modern Pharmaceutics, 4th Ed., Chapters 9 and 10 (Banker & Rhodes, editors, 2002); Lieberman et al., Pharmaceutical Dosage Forms: Tablets (1989); and Ansel, Introduction to Pharmaceutical Dosage Forms 8th Edition (2004).

Various oral dosage forms can be used, including such solid forms as tablets, capsules (e.g., liquid gel capsule and solid gel capsule), granules and bulk powders. Tablets can be compressed, tablet triturates, enteric-coated, sugar-coated, filmcoated, or multiple-compressed, containing suitable binders, lubricants, diluents, disintegrating agents, coloring agents, flavoring agents, flow-inducing agents, and melting agents. Liquid oral dosage forms include aqueous solutions, emulsions, suspensions, solutions and/or suspensions reconstituted from non-effervescent granules, and effervescent preparations reconstituted from effervescent granules, containing suitable solvents, preservatives, emulsifying agents, suspending agents, diluents, sweeteners, melting agents, coloring agents and flavoring agents.

The pharmaceutically-acceptable carriers suitable for the preparation of unit dosage forms for peroral administration is well-known in the art. Tablets typically comprise conventional pharmaceutically-compatible adjuvants as inert diluents, such as calcium carbonate, sodium carbonate, mannitol, lactose and cellulose; binders such as starch, gelatin and sucrose; disintegrants such as starch, alginic acid and croscarmelose; lubricants such as magnesium stearate, stearic acid and talc. Glidants such as silicon dioxide can be used to improve flow characteristics of the powder mixture. Coloring agents, such as the FD&C dyes, can be added for appearance. Sweeteners and flavoring agents, such as aspartame, saccharin, menthol, peppermint, sucrose, and fruit flavors, are useful adjuvants for chewable tablets. Capsules typically comprise one or more solid diluents disclosed above. The selection of carrier components depends on secondary considerations like taste, cost, and shelf stability, which are not critical, and can be readily made by a person skilled in the art.

Peroral compositions also include liquid solutions, emulsions, suspensions, and the like. The pharmaceutically-acceptable carriers suitable for preparation of such compositions are well known in the art. Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, AVICEL RC-591, tragacanth and sodium alginate; typical wetting agents include lecithin and polysorbate 80; and typical preservatives include methyl paraben and sodium benzoate. Peroral liquid compositions may also contain one or more components such as sweeteners, flavoring agents and colorants disclosed above.

Such compositions may also be coated by conventional methods, typically with pH or time-dependent coatings, such that the subject composition is released in the gastrointestinal tract in the vicinity of the desired topical application, or at various times to extend the desired action. Such dosage forms typically include, but are not limited to, one or more of cellulose acetate phthalate, polyvinylacetate phthalate, hydroxypropyl methyl cellulose phthalate, ethyl cellulose, Eudragit coatings, waxes and shellac.

Compositions described herein may optionally include other drug actives.

Other compositions useful for attaining systemic delivery of the subject compounds include sublingual, buccal and nasal dosage forms. Such compositions typically comprise one or more of soluble filler substances such as sucrose, sorbitol and mannitol; and binders such as acacia, microcrystalline cellulose, carboxymethyl cellulose and hydroxypropyl methyl cellulose. Glidants, lubricants, sweeteners, colorants, antioxidants and flavoring agents disclosed above may also be included.

A liquid composition, which is formulated for ophthalmic use, is formulated such that it can be administered to the eye. The comfort may be maximized as much as possible, although sometimes formulation considerations (e.g. drug stability) may necessitate less than optimal comfort. In the case that comfort cannot be maximized, the liquid may be formulated such that the liquid is tolerable to the patient for topical ophthalmic use. Additionally, an ophthalmically acceptable liquid may either be packaged for single use, or contain a preservative to prevent contamination over multiple uses.

For ophthalmic application, solutions or medicaments are often prepared using a physiological saline solution as a major vehicle. Ophthalmic solutions may preferably be maintained at a comfortable pH with an appropriate buffer system. The formulations may also contain conventional, pharmaceutically acceptable preservatives, stabilizers and surfactants.

Preservatives that may be used in the pharmaceutical compositions disclosed herein include, but are not limited to, benzalkonium chloride, PHMB, chlorobutanol, thimerosal, phenylmercuric, acetate and phenylmercuric nitrate. A useful surfactant is, for example, Tween 80. Likewise, various useful vehicles may be used in the ophthalmic preparations disclosed herein. These vehicles include, but are not limited to, polyvinyl alcohol, povidone, hydroxypropyl methyl cellulose, poloxamers, carboxymethyl cellulose, hydroxyethyl cellulose and purified water.

Tonicity adjustors may be added as needed or convenient. They include, but are not limited to, salts, particularly sodium chloride, potassium chloride, mannitol and glycerin, or any other suitable ophthalmically acceptable tonicity adjustor.

Various buffers and means for adjusting pH may be used so long as the resulting preparation is ophthalmically acceptable. For many compositions, the pH will be between 4 and 9. Accordingly, buffers include acetate buffers, citrate buffers, phosphate buffers and borate buffers. Acids or bases may be used to adjust the pH of these formulations as needed.

Ophthalmically acceptable antioxidants include, but are not limited to, sodium metabisulfite, sodium thiosulfate, acetylcysteine, butylated hydroxyanisole and butylated hydroxytoluene.

Other excipient components, which may be included in the ophthalmic preparations, are chelating agents. A useful chelating agent is edetate disodium (EDTA), although other chelating agents may also be used in place or in conjunction with it.

For intravenous administration, the compositions described herein may be dissolved or dispersed in a pharmaceutically acceptable diluent, such as a saline or dextrose solution. Suitable excipients may be included to achieve the desired pH, including but not limited to NaOH, sodium carbonate, sodium acetate, HCl, and citric acid. In various embodiments, the pH of the final composition ranges from 2 to 8, or preferably from 4 to 7. Antioxidant excipients may include sodium bisulfite, acetone sodium bisulfite, sodium formaldehyde, sulfoxylate, thiourea, and EDTA. Other nonlimiting examples of suitable excipients found in the final intravenous composition may include sodium or potassium phosphates, citric acid, tartaric acid, gelatin, and carbohydrates such as dextrose, mannitol, and dextran. Further acceptable excipients are described in Powell, et al., Compendium of Excipients for Parenteral Formulations, PDA J Pharm Sci and Tech 1998, 52 238-311 and Nema et al., Excipients and Their Role in Approved Injectable Products: Current Usage and Future Directions, PDA J Pharm Sci and Tech 2011, 65 287-332, both of which are incorporated herein by reference in their entirety. Antimicrobial agents may also be included to achieve a bacteriostatic or fungistatic solution, including but not limited to phenylmercuric nitrate, thimerosal, benzethonium chloride, benzalkonium chloride, phenol, cresol, and chlorobutanol.

The compositions for intravenous administration may be provided to caregivers in the form of one more solids that are reconstituted with a suitable diluent such as sterile water, saline or dextrose in water shortly prior to administration. In other embodiments, the compositions are provided in solution ready to administer parenterally. In still other embodiments, the compositions are provided in a solution that is further diluted prior to administration. In embodiments that include administering a combination of a compound described herein and another agent, the combination may be provided to caregivers as a mixture, or the caregivers may mix the two agents prior to administration, or the two agents may be administered separately.

The administration period can be a multi-week treatment cycle as long as the inflammation remains under control and the regimen is clinically tolerated. In some embodiments, a single dosage of Plinabulin or other therapeutic agent can be administered once a week, and preferably once on each of day 1 and day 8 of a three-week (21 day) treatment cycle. In some embodiments, a single dosage of Plinabulin or other therapeutic agent can be administered once a week for two weeks, three weeks, four weeks, five weeks, six weeks, seven weeks, or eight weeks, preferably on the first day of each week. In some embodiments, a single dosage of Plinabulin or other therapeutic agent can be administered once a week, twice a week, three times per week, four times per week, five times per week, six times per week, or daily during a one-week, two-week, three-week, four-week, or five-week treatment cycle. The administration can be on the same or different day of each week in the treatment cycle.

In some embodiments, the administration schedule of plinabulin can be the same as that of the checkpoint inhibitor. In some embodiments, the administration schedule of plinabulin can be different from that of the checkpoint inhibitor. In some embodiments, plinabulin and the checkpoint inhibitor (e.g., nivolumab) are both administered every two weeks (e.g., on day 1 and 15 of a 28-day cycle). In some embodiments, the checkpoint inhibitor (e.g., nivolumab) is administered on day 1 and 15 of a 28-day cycle, and plinabulin is administered on day 1, 8, 15, and 22 of a 28-day cycle. In some embodiments, plinabulin and the checkpoint inhibitor (e.g., pembrolizumab) are both administered every three weeks (e.g., day 1 and 22 in a 42-day cycle). In some embodiments, when plinabulin is used in combination with the first and second checkpoint inhibitors, the plinabulin is administered following the same schedule and on the same day of the administration of the first checkpoint inhibitor, while the first checkpoint inhibitor (e.g., nivolumab at 1 mg/kg) and plinabulin are co-administered once every 3 weeks for 4 doses followed by the administration of a second checkpoint inhibitor (e.g., ipilimumab) on the same day, then the first checkpoint inhibitor (e.g., nivolumab at 240 mg per dose) and plinabulin are co-administered every two weeks for two doses without the second checkpoint inhibitor. In some embodiments, plinabulin is administered before the first immune checkpoint inhibitor. In some embodiments, plinabulin is administered shortly after (e.g., about 30 mins, 1hr, 2 hr, or 4 hr) the administration of the first immune checkpoint inhibitor. In some embodiments, plinabulin is administered simultaneously with the first immune checkpoint inhibitor.

The treatment cycle can be repeated as long as the regimen is clinically tolerated. In some embodiments, the treatment cycle is repeated for n times, wherein n is an integer in the range of 2 to 30. In some embodiments, n is 2, 3, 4, 5, 6, 7, 8, 9, or 10. In some embodiments, a new treatment cycle can occur immediately after the completion of the previous treatment cycle. In some embodiments, a new treatment cycle can occur a period of time after the completion of the previous treatment cycle.

### Examples

### Example 1

Plinabulin was tested using Phosphodiesterase assays including PDE3, PDE4, and PDE5 assays. The source for the PDE3 and PDE5 assays were human platelets and for PDE4 assay was human U937 cells. The substrate for all three assays was 1.01 ÙM [.H]cAMP + cAMP. For all three assays, the pre-incubation time was 15 mins, the incubation time was 20 mins, and the pre-incubation and incubation temperature was 25 °C. The quantification method involves quantitation of [³H]Adenosine, and the significant criteria was set for í 50% of max stimulation or inhibition. The test results were shown in Table 1.

**Table 1. Phosphodiesterase bioassay screening**

| | n | Plinabulin concentration | % of inhibition |
|---|---|---|---|
| PDE3 | 2 | 10 µm | 7 |
| PDE4 | 2 | 10 µm | 77 |
| PDE5 | 2 | 10 µm | 25 |

As shown in Table 1, Plinabulin showed better inhibition activity against PDE4 than other PDE3 and PDE5.

### Example 2

Patients received either docetaxel plus plinabulin (DN) or docetaxel alone (75 mg/m²) (D) treatment. Two dosing cohorts were investigated:
1) 30 mg/m² dosing cohort: patients were randomized (1:1) to receive either docetaxel plus plinabulin at 30 mg/m² (DN 30 mg/m² arm) or docetaxel alone (D arm);
2) 20 mg/m² dosing cohort: patients were randomized (2:1) to receive either docetaxel plus plinabulin at 20 mg/m² (DN 20 mg/m² arm) or docetaxel (D arm) alone.

### Dosing Regimen

Patients received therapy on Day 1 and Day 8 in 3-week cycles. Therapy on Day 1 consisted of 75 mg/m² docetaxel administered via intravenous infusion (IV) over 1 hour, followed 2 hours later (from the time the docetaxel infusion began) by placebo (Arm D) or 30 mg/m² or 20 mg/m² plinabulin (Arm DN) administered via intravenous infusion (IV) over 30 minutes. Oral dexamethasone (16 mg) was given the day prior to, the day of and the day following docetaxel infusion (Day 1). Therapy on Day 8 consisted of placebo (Arm D) or 30 mg/m² or 20 mg/m² plinabulin (Arm DN) administered via intravenous infusion (IV) over 30 minutes.

In patients experiencing drug related Grade > 2 treatment emergent adverse events (except alopecia, anorexia, and fatigue) according to the CTCAE (v3.0) treatment may be delayed until the adverse event has recovered to < Grade 1. Safety laboratory tests met the following criteria prior to treatment with docetaxel at the beginning of each subsequent cycle: AST ≤2.5 × ULN, ALT :52.5 × ULN (≤1.5 × ULN if alkaline phosphatase is >=2.5 × ULN); bilirubin < =ULN; hemoglobin >= 9 g/dL, absolute neutrophil count >= 1.5 × 10⁹ /L and platelets >=100 × 10⁹ /L. Dose reductions were implemented for patients who experience recurrent or specific severe toxicities.

The initial dose of plinabulin was 30 mg/m² or 20 mg/m². Dose adjustments depended on observed adverse events. Volume of administration varied based on assigned dose and patient body surface area. The clinical formulation were supplied as a concentrated solution in 40% Solutol^{®} HS-15/60 % propylene glycol in amber vials containing 80 mg of drug in 20 mL (4 mg/mL) and were stored at room temperature. Each vial was designated for single use. The correct volume of drug (at a concentration of 4 mg/mL in the vial) was diluted in dextrose 5% in water (D5W) at a dilution of 1:20 and administered IV peripherally or centrally. Infusion time may be increased as clinically indicated at the direction of the Sponsor. Plinabulin and placebo should be administered within 6 hours of dilution.

The initial dose of docetaxel was 75 mg/m². Dose adjustments depended on observed adverse events. Administration was carried out with a 1 hour IV infusion per institutional protocol at the dose prescribed by this clinical trial protocol. Oral dexamethasone (16 mg) was given the day prior to, the day of and the day following docetaxel infusion (Day 1). A similar corticosteroid premedication regimen was used in accordance with local institutional practices. The dose of dexamethasone or other corticosteroid was appropriately reduced for patients already utilizing corticosteroids.

Baseline Assessments: (within 14 days of start of treatment, i.e., day - 14 to 1) Physical examination, vital signs, ECOG performance status, concomitant medication usage, safety laboratory tests.

Treatment Phase: Safety assessments (including a complete physical examination) were performed prior to study drug infusion. Safety assessments (including complete physical examination) were performed prior to each subsequent cycle (2+). Additionally, the following were evaluated: CBC with differential/platelets and clinical chemistry were performed up to 72 hours prior to Day 1 of each cycle; an additional assessment occurred in Cycle 1/Day 15; Vital signs (heart rate, respiratory rate, blood pressure and temperature) were taken on the days of infusion immediately before and after each study drug infusion and at 30 and 60 minutes following last infusion on the first cycle. During subsequent cycles, vitals were taken prior to and after each infusion during the physical examination.

Assessment of response to treatment occurred during the rest period of the second cycle (and approximately every 2 cycles thereafter).

Treatment continued until there was evidence of progressive disease, unacceptable treatment-related adverse events, then the study was closed, or the patient was withdrawn from the study (either due to withdrawal of consent or Investigator judgment).

ASSESSMENTS OF SAFETY: Adverse events spontaneously declared by the patients or noted during physical examination, vital signs, ECOG performance status and laboratory tests. The results of the treatment emergent adverse events (TEAE) related to dexamethasone are shown in Table 2. Figure 1 illustrates the percentage of patients showing steroid-related effects with plinabulin administration.

**Table 2. Distribution of any dexamethasone-related treatment emergent adverse events**

| | 30 mg/m² cohort | | 20 mg/m² cohort | |
|---|---|---|---|---|
| | DN arm (N=50) | D arm (N=55) | DN arm (N=40) | D arm (N=18) |
| Number of Patients (%) | 25 (50%) | 17 (30.9%) | 15 (37.5%) | 5 (27.8%) |
| D= docetaxel, DN = docetaxel = plinabulin; TEAE=treatment-emergent adverse event | | | | |

Figure 1 showed that plinabulin caused a dose-dependent increase in steroid (dexamethasone)-related adverse events. The steroid related adverse events for patients receiving 30 mg/m² of plinabulin were statistically more significant (p= 0.026) than patients receiving no plinabulin. The results suggested that the PDE4 inhibitory effect of plinabulin can be translated into relevant PDE4 pharmacological effect.

### Example 3

The effects of Plinabulin on Immune-Related AEs with NivolumablPlinabulin combination were studied. In the two phase 1 studies in NSCLC patients, a total of 10 patients were enrolled to date and received Nivolumab (240 mg or 3 mg/kg) in combination with Plinabulin 13.5 mg/m2 (n=3), or 20 mg/m2 (n=5), or 30 mg/m2 (n=2). Two patients had developed grade 1 or grade 2 IR-AEs, without the need of Steroid treatment. No grade 3/4 IR-AEs were observed. The study results showed that plinabulin is a potent PDE4 inhibitor, and clinically exerted 'steroid-like' effects. Therefore, Plinabulin is therefore a viable alternative to steroids. Preliminary clinical data suggested that the addition of Plinabulin to checkpoint inhibitor therapy can prevent IRAEs.

In one phase 1 study, the patients were selected only after they have met at least the following inclusion criteria: 1) Subjects with histologically or cytologically-confirmed metastatic NSCLC whose disease progressed during/after treatment with at least one platinum-containing chemotherapy regimen. 2) At least 1 prior systemic therapy for metastatic disease. Adjuvant chemotherapy or concurrent chemoradiation for early stage disease does not count as prior therapy unless patients progressed within 6 months of completion of chemotherapy. 3) Prior chemotherapy must have been completed at least 4 weeks or at least 5 half-lives (whichever is longer) before study drug administration, and all adverse events have either returned to baseline or stabilized. 4) Prior definitive radiation therapy must have been completed at least 4 weeks before study drug administration. Prior palliative radiotherapy should be completed at least 2 weeks before study drug administration. Whole brain radiation therapy (WBRT), stereotactic radiosurgery (SRS) and focal radiation to the sites of pain or bronchial obstruction will be considered palliative. No radiopharmaceuticals (strontium, samarium) within 8 weeks before study drug administration. 5) Prior major surgery must be completed at least 4 weeks before study drug administration. Prior minor surgery must be completed at least 1 week before study drug administration and subjects should be recovered. Percutaneous biopsies should be completed at least 10 days prior to study drug administration. In this study, Nivolumab (240mg) was administered to the patients through iv on day 1 and 15 until disease progression; and Plinabulin was administered to the patients through iv at three different doses (13.5 mg/m², 20 mg/m², 30 mg/m²) and will be administered at 40 mg/m² for different groups on day 1, 8 and 15 of until disease progression. Courses repeat every 28 days in the absence of disease progression or unacceptable toxicity.

In the other phase 1 study, the patients were selected only after they have met at least the following inclusion criteria: 1) Subjects must have histologically or cytologically-documented stage IIIB or stage IV, recurrent, or metastatic non-small cell lung cancer (NSCLC); 2) Subjects must have received prior platinum doublet based treatment (Up to 2 lines of prior systemic therapy for metastatic disease are permitted; Adjuvant chemotherapy or concurrent chemoradiation for early stage disease does not count as prior therapy unless subject progressed within 6 months of completion of regimen; Patients with known activating mutations in epidermal growth factor receptor (EGFR), or known translocation in anaplastic lymphoma kinase (ALK) or ROS-1 are eligible provided they have progressed on or were intolerant to Food and Drug Administration (FDA) approved targeted therapy); 3) Subjects must have an Eastern Cooperative Oncology Group (ECOG) performance status of 0 to 2; 4) Subjects, including those in the dose-escalation portion of the study, must have measurable disease per Response Evaluation Criteria in Solid Tumors (RECIST) 1.1 criteria; imaging must be within 28 days of trial enrollment (Target lesions may be located in a previously irradiated field if there is documented (radiographic) disease progression in that site prior to trial enrollment); 5) Absolute neutrophil count (ANC) >= 1U00/mm^3; 6) Platelets >= 75,000/dL; 7) Hemoglobin >= 9 g/dL; 8) Total bilirubin =< 1.5 mg/dL x upper limit of normal (ULN) (except subjects with Gilbert syndrome who can have total bilirubin =< 3.0 mg/dL); 9) Serum creatinine =< 1.5 mg/dL or creatinine clearance >= 60 mL/min; 10) Alanine aminotransferase (ALT) and aspartate aminotransferase (AST) =< 2.5 times the upper limit of normal if no liver involvement or =< 5 times the upper limit of normal with liver involvement. During the study, patients received plinabulin IV over 30 minutes and nivolumab IV (3mg/kg) over 60 minutes on days 1 and 15. Courses repeat every 28 days in the absence of disease progression or unacceptable toxicity. Two doses of plinabulin (20 mg/m² and 30 mg/m²) were tested. Percentage of patients experiencing grade 3 or higher severity of adverse events (adverse events graded using the National Cancer Institute Common Toxicity Criteria version 4.0) was studied.

The subject matter encompassed by the following numbered clauses also forms part of the present invention, optionally in combination with the subject matter described above and/or defined in the clauses.

### CLAUSES

1. A method of treating or preventing immunotherapy related adverse event in a subject, comprising administering an effective amount of plinabulin to the subject in need thereof, wherein the subject is administered one or more immune checkpoint inhibitor.
2. The method of clause 1, wherein the one or more checkpoint inhibitor is selected from the group consisting of a PD-1 inhibitor, a PD-L1 inhibitor, a PD-L2 inhibitor, a PD-L3 inhibitor, a PD-L4 inhibitor, a CTLA-4 inhibitor, a LAG3 inhibitor, a B7-H3 inhibitor, a B7-H4 inhibitor, a KIR inhibitor and a TIM3 inhibitor.
3. The method of clause 1 or 2, wherein the immune checkpoint inhibitor is a PD-1 inhibitor.
4. The method of clause 1 or 2, wherein the immune checkpoint inhibitor is a PD-L1 inhibitor.
5. The method of clause 1 or 2, wherein the immune checkpoint inhibitor is a CTLA-4 inhibitor.
6. The method of clause 1, wherein the subject is administered a first immune checkpoint inhibitor and a second immune checkpoint inhibitor, and wherein the first immune checkpoint inhibitor is different from the second immune checkpoint inhibitor.
7. The method of clause 6, wherein the first and the second immune checkpoint inhibitor is independently an inhibitor selected from the group consisting of a PD-1 inhibitor, a PD-L1 inhibitor, a PD-L2 inhibitor, a PD-L3 inhibitor, a PD-L4 inhibitor, a CTLA-4 inhibitor, a LAG3 inhibitor, a B7-H3 inhibitor, a B7-H4 inhibitor, a KIR inhibitor and a TIM3 inhibitor.
8. The method of clause 7, wherein the first checkpoint inhibitor is a PD-1 inhibitor or a PD-L1 inhibitor, and the second checkpoint inhibitor is a CTLA-4 inhibitor.
9. The method of clause 1, wherein the immunotherapy is a monotherapy that comprises administering a single checkpoint inhibitor selected from the group consisting of a PD-1 inhibitor, a PD-L1 inhibitor, a PD-L2 inhibitor, a PD-L3 inhibitor, a PD-L4 inhibitor, a CTLA-4 inhibitor, a LAG3 inhibitor, a B7-H3 inhibitor, a B7-H4 inhibitor, a KIR inhibitor and a TIM3 inhibitor.
10. The method of clause 9, wherein the checkpoint inhibitor is a PD-1 inhibitor.
11. The method of clause 9, wherein the checkpoint inhibitor is a PD-L1 inhibitor.
12. The method of clause 9, wherein the checkpoint inhibitor is a CTLA-4 inhibitor.
13. The method of clause 1, wherein the immunotherapy is a combination therapy, and wherein the combination therapy comprises administering two or more checkpoint inhibitors selected from the group consisting of a PD-1 inhibitor, a PD-L1 inhibitor, a PD-L2 inhibitor, a PD-L3 inhibitor, a PD-L4 inhibitor, a CTLA-4 inhibitor, a LAG3 inhibitor, a B7-H3 inhibitor, a B7-H4 inhibitor, a KIR inhibitor and a TIM3 inhibitor.
14. The method of clause 13, wherein the combination therapy comprises administering a PD-1 inhibitor and a CTLA-4 inhibitor.
15. The method of clause 13, wherein the combination therapy comprises administering a PD-L1 inhibitor and a CTLA-4 inhibitor.
16. The method of any one of clauses 13 to 15, wherein the amount of plinabulin is effective to inhibit PDE4 activity without reducing a vascular proliferation or density.
17. The method of any one of clauses 13 to 16, wherein the amount of plinabulin is in the range of about 1 mg to about 100 mg.
18. The method of any one of clauses 1 to 17, wherein the immunotherapy related adverse event is a grade 3/4 adverse event.
19. The method of any one of clauses 1 to 18, comprising identifying a subject at risk of developing a grade 3/4 immunotherapy related adverse event.
20. The method of any one of clauses 1 to 19, wherein the immunotherapy related adverse event is selected from the group consisting of Pneumonitis, Colitis, Hepatitis, Endocrinopathies, Nephritis and Renal Dysfunction, Skin Adverse Reactions, and Encephalitis
21. The method of clause 20, wherein the immunotherapy related adverse event is pancreatitis, and wherein the immunotherapy comprises administering a PD-1 inhibitor and a CTLA-4 inhibitor.
22. The method of any one of clauses 1 to 20, wherein the immunotherapy related adverse event is the immunotherapy induced inflammation.
23. The method of clause 22, wherein the immunotherapy induced inflammation is selected from the group consisting of pancreatitis, pneumonitis, colitis, hepatitis, nephritis and renal dysfunction, hypothyroidism and hyperthyroidism, uveitis, demyelination, autoimmune neuropathy, adrenal insufficiency, facial and abducens nerve paresis, hypophysitis, diabetic ketoacidosis, hypopituitarism, Guillain-Barré syndrome, myasthenic syndrome, hypothysitis, thyroiditis, type 1 diabetes mellitus, arthritis, exfoliative dermatitis, bullous pemphigoid, myositis, myasthenia gravis, vasculitis, hemolytic anemia, partial seizures arising in a patient with inflammatory foci in brain parenchyma, dermatitis, rash, pruritus, meningitis, sarcoidosis, pericarditis, fatal myocarditis, angiopathy, temporal arteritis, vasculitis, polymyalgia rheumatica, conjunctivitis, blepharitis, episcleritis, scleritis, iritis, leukocytoclastic vasculitis, erythema multiforme, psoriasis, arthritis, autoimmune thyroiditis, neurosensory hypoacusis, autoimmune central neuropathy, myositis, polymyositis, and ocular myositis, and hemolytic anemia.
24. The method of any one of clauses 1 to 23, further comprising identifying a subject having the immunotherapy related adverse event prior to administering an effective amount of plinabulin to the subject.
25. The method of any one of clauses 1 to 24, further comprising identifying a subject having a grade 3/4 immunotherapy related adverse event prior to administering an effective amount of plinabulin to the subject
26. The method of any one of clauses 1 to 25, wherein the incidence of immunotherapy related adverse event is reduced by at least 10%.
27. The method of any one of clauses 1 to 26, wherein the severity of immunotherapy related adverse event is reduced by at least 10%.
28. A method of reducing an incidence of an immunotherapy related adverse event comprising administering an effective amount of plinabulin to the subject in need thereof, wherein the subject is administered one or more immune checkpoint inhibitor.
29. A method of shortening a duration of an immunotherapy related adverse event comprising administering an effective amount of plinabulin to the subject in need thereof, wherein the subject is administered one or more immune checkpoint inhibitor.
30. The method of any one of clauses 1-29, wherein the subject also receives a radiation therapy.
31. A method of treating or preventing an inflammatory skin or joint disorder in a subject, comprising topically administering an effective amount of plinabulin to the subject in need thereof.
32. The method of clause 31, wherein the skin or joint disorder is psoriasis.
33. The method of clause 31, wherein the skin or joint disorder is arthritis.
34. The method of clause 31, wherein the skin or joint disorder is rheumatoid arthritis.
35. The method of clause 31, wherein the skin or joint disorder is selected from ankylosing spondylitis, psoriatic arthritis, sarcoidosis, systemic lupus erythematosus, or atopic dermatitis.
36. A method of treating or preventing an inflammatory disease in a subject, comprising administering an effective amount of plinabulin to the subject in need thereof, wherein the inflammatory disease is selected from the group consisting of chronic articular rheumatism, chronic obstructive pulmonary disease, asthma, ankylosing spondylitis, psoriatic arthritis, sarcoidosis, systemic lupus erythematosus, inflammatory bowel disease, atopic dermatitis, and multiple sclerosis.
37. The method of clause 36, wherein the plinabulin is administered parenterally.
38. The method of clause 36, wherein the plinabulin is administered orally.
39. The method of clause 36, wherein the plinabulin is administered topically.
40. A method of treating or preventing chronic obstructive pulmonary disease or asthma in a subject comprising administering an effective amount of plinabulin to the subject in need thereof through an inhaler.
41. A topical formulation, comprising plinabulin at a concentration effective to inhibit PDE4 activity without reducing vascular proliferation or density.
42. A topical formulation, comprising plinabulin at a concentration in the range of about 0.1% to about 10% by weight of the total formulation.
43. The formulation of clause 41 or 42, wherein the concentration of plinabulin is in the range of about 0.5% to about 5% by weight of the total formulation.
44. The formulation of any one of clauses 41 to 43, further comprising one or more excipient.
45. The formulation of any one of clauses 41 to 44, further comprising one or more ingredients selected from white petrolatum, propylene glycol, mono- and di-glycerides, paraffin, butylated hydroxytoluene, or edetate calcium disodium.
46. The formulation of any one of clauses 41 to 45, further comprising Polyoxyl 15 hydroxystearate.
47. The formulation of any one of clauses 41 to 46, wherein the formulation is in the form of cream, ointment, gel, emulsion, or suspension.

## Claims

1. Plinabulin for use in treating or preventing an inflammatory skin or joint disorder.

2. Plinabulin for use according to claim 1, wherein the inflammatory skin or joint disorder is selected from the group consisting of psoriasis, arthritis, rheumatoid arthritis, ankylosing spondylitis, psoriatic arthritis, sarcoidosis, systemic lupus erythematosus, and atopic dermatitis.

3. Plinabulin for use in treating or preventing inflammatory disease, wherein the inflammatory disease is selected from the group consisting of chronic articular rheumatism, chronic obstructive pulmonary disease, asthma, ankylosing spondylitis, psoriatic arthritis, sarcoidosis, systemic lupus erythematosus, inflammatory bowel disease, atopic dermatitis, and multiple sclerosis.

4. Plinabulin for use according to claim 3, wherein plinabulin is for administration parenterally, orally or topically.

5. Plinabulin for use in treating or preventing chronic obstructive pulmonary disease or asthma, wherein an effective amount of plinabulin is for administration to the subject in need thereof through an inhaler.

6. A topical formulation, comprising plinabulin at a concentration effective to inhibit PDE4 activity without reducing vascular proliferation or density.

7. A topical formulation, comprising plinabulin at a concentration in the range of about 0.1% to about 10% by weight of the total formulation.

8. The topical formulation of claim 7, wherein the concentration of plinabulin is in the range of 0.5% to about 5% by weight of the total formulation.

9. The formulation of any one of claims 7 to 8, further comprising one or more excipient.

10. The formulation of any one of claims 7 to 9, further comprising one or more ingredients selected from white petrolatum, propylene glycol, mono- and di-glycerides, paraffin, butylated hydroxytoluene, or edetate calcium disodium.

11. The formulation of any one of claims 7 to 10, further comprising Polyoxyl 15 hydroxystearate.

12. The formulation of any one of claims 7 to 11, wherein the formulation is in the form of cream, ointment, gel, emulsion, or suspension.
